(19) Europäisches Patentamt / European Patent Office / Office européen des brevets

(11) **EP 4 200 862 B1**

(12) **EUROPEAN PATENT SPECIFICATION**

(45) Date of publication and mention
of the grant of the patent:
**10.12.2025 Bulletin 2025/50**

(21) Application number: **21735891.0**

(22) Date of filing: **18.06.2021**

(51) International Patent Classification (IPC):
**G16B 40/20** (2019.01)        **G16B 5/00** (2019.01)

(52) Cooperative Patent Classification (CPC):
**G16B 5/00; G16B 40/20**

(86) International application number:
**PCT/EP2021/066541**

(87) International publication number:
**WO 2022/106074 (27.05.2022 Gazette 2022/21)**

(54) **METHOD AND SYSTEM FOR QUANTIFYING CELLULAR ACTIVITY FROM HIGH THROUGHPUT SEQUENCING DATA**

VERFAHREN UND SYSTEM ZUR QUANTIFIZIERUNG DER ZELLAKTIVITÄT AUS HOCHDURCHSATZSEQUENZIERUNGSDATEN

PROCÉDÉ ET SYSTÈME DE QUANTIFICATION D'ACTIVITÉ CELLULAIRE À PARTIR DE DONNÉES DE SÉQUENÇAGE À HAUT DÉBIT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.11.2020 EP 20208656**

(43) Date of publication of application:
**28.06.2023 Bulletin 2023/26**

(73) Proprietor: NEC Corporation
Minato-ku
Tokyo 108-8001 (JP)

(72) Inventors:
• **PILEGGI, Giampaolo**
69115 Heidelberg (DE)
• **MALONE, Brandon**
69115 Heidelberg (DE)

(74) Representative: **Ullrich & Naumann PartG mbB**
**Schneidmühlstrasse 21**
**69115 Heidelberg (DE)**

(56) References cited:
• **KYU-TAE KIM ET AL: "Single-cell mRNA sequencing identifies subclonal heterogeneity in anti-cancer drug responses of lung adenocarcinoma cells", GENOME BIOLOGY, BIOMED CENTRAL LTD, vol. 16, no. 1, 19 June 2015 (2015-06-19), pages 127, XP021228165, ISSN: 1465-6906, DOI: 10.1186/ S13059-015-0692-3**
• **FROST F. GRAEME ET AL: "Pan-cancer RNA-seq data stratifies tumours by some hallmarks of cancer", JOURNAL OF CELLULAR AND MOLECULAR MEDICINE, vol. 24, no. 1, 15 November 2019 (2019-11-15), RO, pages 418 - 430, XP055841188, ISSN: 1582-1838, DOI: 10.1111/jcmm.14746**
• **FAN JEAN ET AL: "Characterizing transcriptional heterogeneity through pathway and gene set overdispersion analysis", NATURE METHODS, vol. 13, no. 3, 1 March 2016 (2016-03-01), New York, pages 241 - 244, XP055841194, ISSN: 1548-7091, DOI: 10.1038/nmeth.3734**
• **MATHIAS UHLEN ET AL: "A pathology atlas of the human cancer transcriptome", SCIENCE, vol. 357, no. 6352, 18 August 2017 (2017-08-18), US, pages 1 - 13, XP055693994, ISSN: 0036-8075, DOI: 10.1126/science.aan2507**

**Description**

The present invention relates to a computer-implemented method and a processing system for patient stratification using high throughput sequencing data.

During the latest years, high throughput sequencing methods (such as single-cell sequencing, sc-seq) have been developed, which allow the users to analyze in detail the entirety of the gene expression (transcriptomes), at the resolution of the single cell. Consequently, such methods constitute a valuable technology for investigating tumoral heterogeneity (that is, the variability of expressed genes in the different tumoral cells of a patient), in order to predict treatment outcomes for the patients before starting the therapy.

Although high throughput (and mostly sc-seq) data can provide many insights on tumors, such data are affected by different forms of noise, such as batch effects and dropout events. For instance, Batch effects may occur when different expression values of the genes in samples taken from the same patient are observed. Dropouts denote the presence of ambiguous missing values in the data, where it is unknown if they derive from the non-expression of a gene or the non-detection of its expression value. The most important problem, however, is that, when the data are used "as is" to perform predictions on the response of the samples (e.g., the cells of a patient, in the case of sc-seq) to a specific treatment, results are really poor, making it impossible to make effective therapy recommendations.

It is therefore an object of the present invention to improve and further develop a method and a processing system of the initially described type for patient stratification using high throughput sequencing data in such a way that a higher level of information for ML-based predictions is provided.

In accordance with embodiments of the invention, the aforementioned object is accomplished by a computer-implemented method comprising the features of claim 1.

Furthermore, the aforementioned object is accomplished by a processing system comprising the features of independent claim 6.

According to embodiments of the invention it has been recognized that the above mentioned object can be accomplished by a method and a system using an algorithm for producing low-level representations of noisy cell expressions obtained from high throughput sequencing (e.g. singleRNA-cell sequencing). In particular, embodiments of the invention relate to a method that represents each cell in the data as the distance between its embedding and the centroids of gene clusters (gene modules), which are obtained by appropriately clustering multimodal information, e.g. coming from domain knowledge. The method may also include specific functions for imputing missing values from the raw high throughput data. One important application deriving from this representation is patient stratification: by using activation vectors as input for a Machine Learning algorithm, it is possible to predict the response of the cells (and consequently, of the patient) to a specific therapy, by using the activity level of the gene modules as highly discriminative features.

According to embodiments of the invention, an important aspect of the method consists of the activation vector, AV, construction: The construction process may start from a Gene Regulatory Network (GRN), which is a knowledge graph whose nodes are genes, and to which additional features are associated, which may be obtained from domain knowledge. Gene Modules (GMs) may be obtained by clustering the embedding of nodes of said GRN. By linearly combining the high throughput sample data with the embeddings of the genes, the single sample can be represented in an embedded form. Last, since the GM clusters and the samples are in the same embedding space, it is possible to calculate the distance of each sample (or cell, in the case of sc-seq) from each centroid of the different GMs, thus obtaining the AV representation.

According to an embodiment of the processing system for quantifying cellular activity from high throughput sequencing data, the present invention proposes a multistage computational pipeline to stratify patients based on scRNA-seq data. The pipeline first uses graph convolutional neural networks (GCNN) in order to combine multiple modalities of domain knowledge, including gene regulatory networks and gene annotations, to learn low-dimensional representations of genes; the genes are further clustered into functionally-relevant gene modules. Second, a collaborative filtering approach is used to remove artifacts and other sources of noise from the scRNA-seq data. The cleaned scRNA-seq data is then combined with low-dimensional gene module representations to produce a low-dimensional representation for each cell. A supervised machine learning model is trained to predict whether each cell came from a sample which will respond to a particular treatment or not. Those predictions are finally combined to stratify samples into responders and non-responders.

According to embodiments of the invention, the sequencing data includes single RNA-cell sequencing data, such that each sample of the sequencing data is a single cell.

According to embodiments of the invention, embedding the samples of the sequencing data into the multimodal knowledge graph may include linearly combining the sequencing data with the embeddings of the genes.

According to embodiments of the invention, the method may further comprise using a graph convolutional neural network, GCNN, to create the embeddings of each gene based on the GRN with the gene annotations. The embeddings may then be clustered and provided as the GMs.

According to embodiments of the invention, the method may further comprise, prior to generating the activation vectors, applying a collaboration filtering algorithm to remove dropout values and other sources of noise from the high throughput sequencing data.

According to the invention, the method comprises using the activation vectors as input for training a machine learning algorithm to predict a response of individual cells to an applied drug.

According to the invention, the method comprises using the trained machine learning algorithm to predict a label 'responder' or 'non-responder' for each cell of the sequencing data. In this context, a voting process is executed that establishes the total response of a patient to a specific drug with a confidence score, wherein the fraction of cells predicted to respond to the drug represents the confidence that the patient will respond to the drug.

According to embodiments of the invention, the method may further comprise providing as output one or more of the most important activation vectors used in the prediction as an explanation for the obtained results.

According to embodiments of the invention, the processing system may be integrated in a platform to be used, e.g., in hospitals or insurance companies. Alternatively, the processing system can be used in bioinformatics pipelines and products. In any case, before being operational, the processing system requires high throughput training data with known response to each drug of interest. Thus, it may not be effective in a purely prospective study in which outcomes are unknown. Furthermore, according to embodiments, external domain knowledge is needed for the generation of valid Gene Modules.

There are several ways how to design and further develop the teaching of the present invention in an advantageous way. To this end it is to be referred to the dependent claims on the one hand and to the following explanation of preferred embodiments of the invention by way of example, illustrated by the figure on the other hand. In connection with the explanation of the preferred embodiments of the invention by the aid of the figure, generally preferred embodiments and further developments of the teaching will be explained. In the drawing

Fig. 1     is a flowchart for obtaining an Activation Vector representation for patent stratification according to an embodiment of the present invention,

Fig. 2     is a schematic view illustrating the identification of gene clusters from external information embeddings in accordance with an embodiment of the present invention,

Fig. 3     is a schematic view illustrating the imputation of dropout expression values in accordance with an embodiment of the present invention,

Fig. 4     is a schematic view illustrating a representation of individual cells clustered through hierarchical clustering in accordance with an embodiment of the present invention,

Fig. 5     is a schematic view illustrating the structure of a system for performing patient stratification from single-cell data in accordance with an embodiment of the present invention,

Fig. 6     is a diagram showing prediction results (true positive rate vs false positive rate) obtained by a method in accordance with embodiments of the present invention in contrast to prior art methods.

Fig. 7     is a diagram showing gene modules clustering in accordance with an embodiment of the present invention,

Fig. 8     is a diagram illustrating imputation benchmarking by comparing the imputed values of two replicate cells in accordance with an embodiment of the present invention,

Fig. 9     is a diagram illustrating hierarchical clustering of activation vectors in accordance with an embodiment of the present invention,

Fig. 10    is a diagram showing activation vector distributions by gene module in accordance with an embodiment of the present invention,

Fig. 11    is a diagram showing gene expression distributions for top-ranked genes in accordance with an embodiment of the present invention,

Fig. 12    is a diagram showing a pipeline for a patient stratification process in accordance with an embodiment of the present invention,

Fig. 13    is a diagram showing boxplots that indicate gene module importances for patient stratification in accordance with an embodiment of the present invention, and

Fig. 14   depicts tables showing the gene ontologies contained in the different clusters in accordance with an embodiment of the present invention.

Tumor heterogeneity poses a significant challenge in the treatment of cancers. In particular, a high degree of intra-tumor heterogeneity can result in subpopulations of cells with very different genetic signatures in the tumor microenvironment (TME). As a result, even patients with seemingly-similar conditions may respond very differently to the same treatment. Thus, it is extremely important to analyze the specific gene signatures that characterize the tumor heterogeneity itself in order to understand which patients will respond to a specific treatment. In the context of the present disclosure, this grouping of patients into responders and non-responders is referred to as patient stratification.

High-throughput, single-cell sequencing methods, such as single-cell RNA sequencing (scRNA-seq), enable detailed analysis of the entirety of the genetic profile at the resolution of the single cell. Consequently, sc-RNAseq constitutes a valuable technology for investigating the heterogeneity inside the TME. However, analyzing scRNA-seq data is a challenging task due to various artifacts, such as batch effect, dropout events, technical noise, as well as statistical challenges such as the curse of dimensionality. Dropout effects and technical noise in particular are very frequent, and both result in zero observed expression for specific transcripts. This makes it very difficult to discern whether the observations comes from non-expression or non-detection of the transcript.

A variety of imputation methods have been developed to account for dropout and other artifacts. Conceptually, these algorithms borrow information across similar transcripts and cells to make up for the missing values: while MAGIC (for reference, see D. van Dijk et al.: Recovering gene interactions from single-cell data using data diffusion. Cell 174(3), 716-72927 (2018)) and SAVER (for reference, see M. Huang et al.: SAVER: gene expression recovery for single-cell RNA sequencing. Nature Methods 15, 539-542 (2018)) use a Markov transition matrix and gene-to-gene relationship, respectively, in order to remap all the values (not only the missing ones). Another approach, called scImpute (for reference, see W.V. Li et al.: An accurate and robust imputation method scImpute for single-cell RNA-seq data. Nature Communications 9(997) (2018)) relies on a mixture model to predict the most likely dropouts and only corrects them. Other techniques rely on deep learning (DL) algorithms to perform their tasks. DCA (for reference, see G. Eraslan et al.: Single-cell RNA-seq denoising using a deep count autoencoder. Nature Communications 10(390) (2019)) uses an autoencoder for retrieving non-linear relations among genes, resulting in a more accurate imputation, while GraphSCI (for reference, see j. Rao et al.: Imputing Single-cell RNA-seq data by combining Graph Convolution and Autoencoder Neural Networks (2020). bioRxiv 2020.02.05.935296) uses a gene-gene network, and the expression values extracted from it, to perform the imputation by using a graph convolutional network and an autoencoder. Such imputation techniques are important for preparing data for further analysis, but they do not aim to address the patient stratification problem.

Another notable use of DL (Deep Learning) is for unsupervised analysis purposes. Autoencoders (for reference, see X. Li et al: Deep learning enables accurate clustering with batch effect removal in single-cell RNA-seq analysis", Nature Communications 11(2338) (2020)) were used to perform dimensionality reduction and reduction of the batch effect noise, in order to obtain meaningful clusters by taking into account the non-linearity in the data, with the aim of discovering specific genetic signatures, and for removing batch effect noise. Other works used DL approaches in order to analyze intratumoral heterogeneity. For example, Sharma et al.: Longitudinal single-cell RNA sequencing of patient-derived primary cells reveals drug-induced infidelity in stem cell hierarchy. Nature Communications 9(4931) (2018), identify cancer-resistant subpopulations by characterizing different stages of tumoral evolution. However, unsupervised analysis approaches have limited use for patient stratification since they do not aim to predict outcomes for unseen examples.

Several strategies have also focused on characterizing individual cells by directly incorporating gene sets rather than traditional differential expression of individual genes. For example, PAGODA (for reference, see J. Fan, J. et al.: Characterizing transcriptional heterogeneity through pathway and gene set overdispersion analysis. Nature Methods 13, 241-244 (2016)) uses gene sets based on Gene Ontology (GO) and other annotated pathways, as well some identified de novo from the scRNA-seq expression; it then characterizes cells according to gene sets with overdispersed expression values. DCell (for reference, see J. Ma et al.: Using deep learning to model the hierarchical structure and function of a cell. Nature Methods 15, 290-298 (2018)) builds a neural network structure based on hierarchical relationships among annotations, such as the GO, tailored specifically for yeast; the network is then trained to predict cell viability based on single- and double-gene-deletion genotype data. Expression of sets of "gene modules" have been used to hierarchically cluster tumor samples (for reference, see F.G. Frost et al.: Pan-cancer RNA-seq data stratifies tumours by some hallmarks of cancer. Journal of Cellular and Molecular Medicine 24, 418-430 (2020)); however, this approach is not designed to stratify unseen samples and cannot make new predictions.

Recently, a variety approaches have been applied to the patient stratification problem. While much of this work focuses on general electronic health records (for a recent review, see B. Shickel et al.: Deep EHR: A survey of recent advances in deep learning techniques for electronic health record (EHR) analysis. IEEE Journal of Biomedical and Health Informatics 22(5), 1589-1605 (2018)), other approaches have also been applied. For instance, D. Toro-Domínguez et al.:

Differential treatments based on drug-induced gene expression signatures and longitudinal systemic lupus erythematosus stratification. Scientific Reports 9(15502) (2019) devised a patient stratification approach based on the correlation of clinical indicators and neutrophil count across several time points. Fröhlich et al.: Premenopausal breast cancer: potential clinical utility of a multi-omics based machine learning approach for patient stratification. EPMA Journal 9, 175-186 (2018) stratified breast cancer patients into risk groups from proteomic and metabolomics data using gradient boosting, while other machine learning-based patient stratification approaches have relied on feature selection approaches (as described in P. Jeyananthan et al.: Classification and regression analysis of lung tumors from multi-level gene expression data. Proceedings of the International Joint Conference on Neural Networks (2019)). Clustering-based approaches (for reference, see Y. Jang et al.: CaPSSA: visual evaluation of cancer biomarker genes for patient stratification and survival analysis using mutation and expression data. Bioinformatics 35(24), 5341-5343 (2019)) and statistical model-based, differential expression analysis (for reference, see F.J. Campos-Laborie et al.: DECO: decompose heterogeneous population cohorts for patient stratification and discovery of sample biomarkers using omic data profiling. Bioinformatics 35(19), 3651-3662 (2019)) have also been used for patient stratification. However, all of these approaches base their stratification on the expression of single genes (or proteins, metabolites, etc.). Despite significant advances in sequencing and other technology, reproducibility remains low across platforms and testing sites (for reference, see L. Yu: RNA-Seq reproducibility assessment of the sequencing quality control project. Cancer informatics 19, 1-5 (2020)). Thus, the generalizability of methods which rely on such individual measurements is not clear.

According to another approach (for reference, see K.-T. Kim et al.: Single-cell mRNA sequencing identifies subclonal heterogeneity in anti-cancer drug responses of lung adenocarcinoma cells. Genome Biology 16(127) (2015)), a risk score predictive of drug response in lung adenocarcinoma (LUAD) has been derived based on the expression of a known panel of genes. In the context of this work, it has also been demonstrated that the risk score could be meaningfully applied to individual cells. According to the inventors' assessment, this is the most related approach to the proposed patient stratification strategy described in the present disclosure. As described further below, the patient stratification solution according to the present invention is empirically compared to a similar approach and it is demonstrated that the solution according to the present invention outperforms it.

Embodiments of the present invention provide a computational pipeline based on a graph neural network approach that combines scRNA-seq expression data with prior knowledge in order to perform patient stratification. In contrast to typical differential expression analysis which focus on fold change of specific genes, embodiments of the invention achieve patient stratification by evaluating the activity level of gene modules across all of their cells. Embodiments of the present invention implement a graph neural network-based approach to combine information of a gene regulatory network (GRN) with gene annotations into a single vector representation, or embedding; the embeddings may then be clustered in order to obtain Gene Modules (GMs), which, in the context of the present disclosure, are understood to represent groups of similar genes. According to a further embodiment, the present invention provides a collaborative filtering approach to remove artifacts and noise in scRNA-seqexpression data.

Generally, embodiments of the present invention can thus provide an interpretable patient stratification approach that accounts for tumor heterogeneity using the gene modules and cleaned scRNA-seq expression data. Embodiments of the present invention outperform existing approaches. In particular, as has been empirically evaluated by the inventors and as will be shown below, embodiments of the invention significantly outperform baselines for stratifying Multiple Myeloma patients into responders and non-responders for the drug panobinostat.

According to an embodiment, the present application provides a method for quantifying cellular activity from high throughput sequencing data, the method including the following steps/aspects:

> 1. The usage of a multimodal knowledge base, by combining a Gene-Regulatory Network, GRN (graph in which each node is a gene) with annotations coming from domain knowledge.
> 2. The embedding process of the GRN and the clustering of its nodes, in order to obtain Gene Modules and the respective cluster centroids.
> 3. The acquisition of high-throughput data in the form of Sample x Genes matrix. In the case of sc-seq, then each cell corresponds to a sample.
> 4. Combining the sequencing data to the gene embedding (from Step 2) to produce an embedding of the samples.
> 5. The creation of Activation Vectors based on the distance between the samples and the Gene Modules centroids. In other words, a distance-based representation, in which the distance between clusters of embedded genes (a measure of genetic functionality) and the embedding of cells (a measure of genetic activity), is used to produce a representation of the cells of a patient as Activation Vectors, that is, the activity level of clusters of genes.

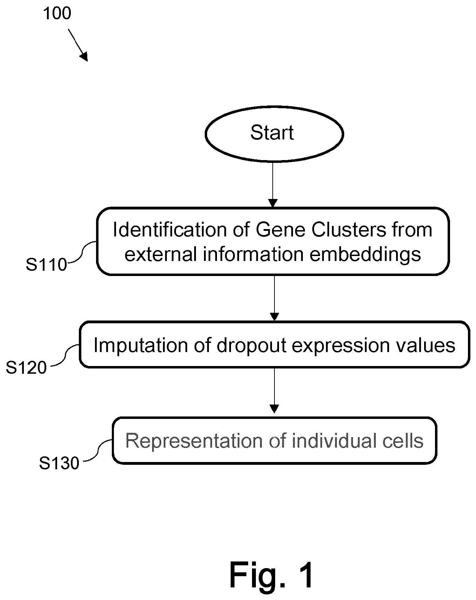

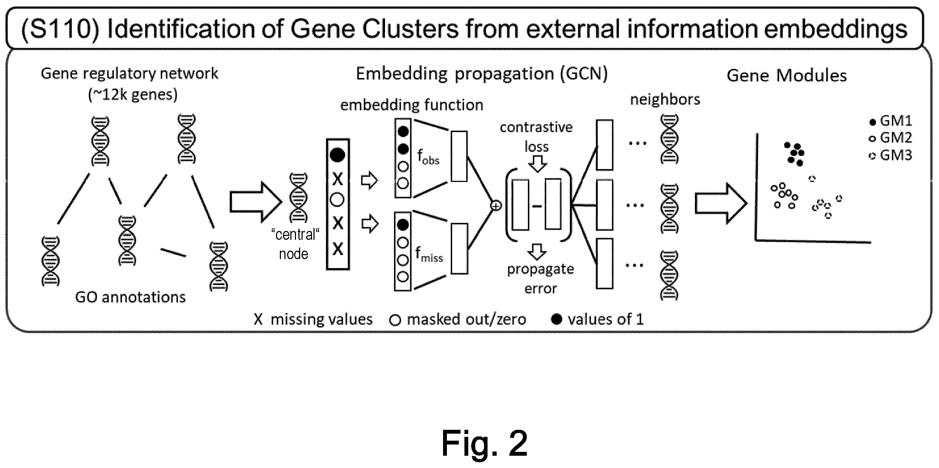

According to an embodiment, the present invention provides a computational pipeline 100 for patient stratification that consists of three main phases/components, wherein an overview of the full flowchart describing the process is depicted in Fig. 1. Figs. 2-4 illustrate the individual phases/components in more detail.

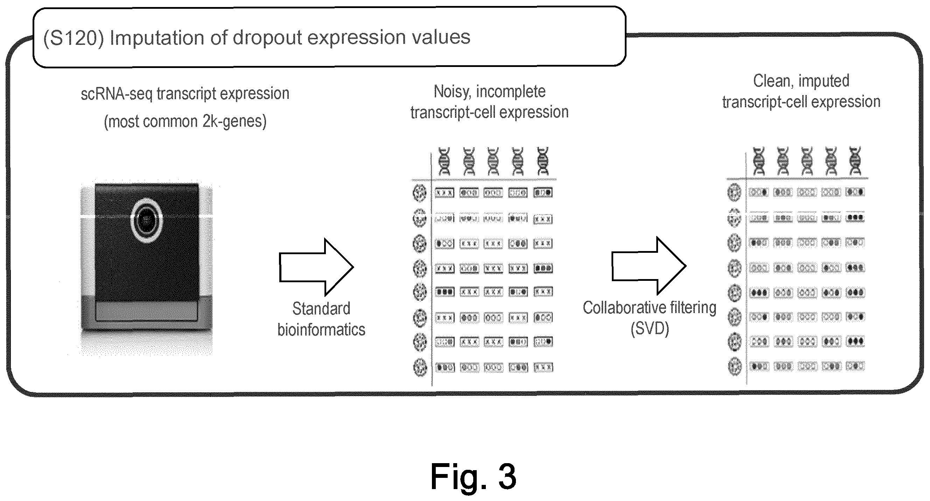

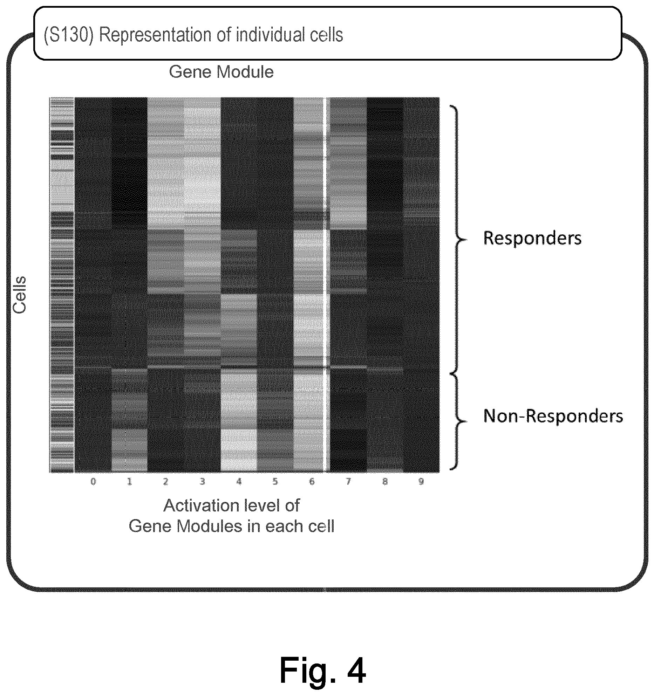

First, as shown at step S110 in Fig. 1 and in further detail in Fig.2, the patient stratification pipeline 100 uses external knowledge from a gene regulatory network (GRN) and gene annotations in order to create gene modules (GMs) with a graph neural network. The GMs are created in such a way that each GM comprises a group of similar genes. Second, as shown at step S120 in Fig. 1 and in further detail in Fig. 3, the patient stratification pipeline 100 uses bioinformatics and collaborative filtering methods (e.g. singular value decomposition (SVD) filtering) in order to remove artifacts and noise from scRNA-seq data. Third, as shown at step S130 in Fig. 1 and in further detail in Fig. 4, the patient stratification pipeline 100 creates, based on the inputs from steps S110 and S120, GM activation vectors for each cell and trains a machine learning model to predict drug response for individual cells. These individual cellular predictions are finally combined in order to stratify patients into responders and non-responders. The following sections describe each step in detail.

**Learning gene embeddings**

In an embodiment, learning gene embeddings is performed by using a graph convolutional neural network (GCNN) in order to create dense vector representations, i.e. embeddings, of each gene based on a GRN and gene annotations. The embeddings are then clustered, and these clusters are taken as the gene modules GMs.

Each gene in the GRN may be associated with a set of gene annotations, which are treated as bag-of-word features. According to an embodiment, the set of gene annotations may include, e.g. Gene Ontology terms. Then, the representations are learned, for instance by using the embedding propagation (EP) algorithm as described in García-Durán, A., Niepert, M.: Learning graph representations with embedding propagation. *Advances in Neural Information Processing Systems* 30 (2017). Briefly, EP is a GCNN that learns embeddings for each node feature by minimizing the difference between its embedding and those obtained from neighboring nodes in the GRN.

More formally, the learning framework of EP entails two phases. First, the EP algorithm may learn an embedding for each of the annotations, as well as for each gene identity, by passing messages along the edges of the GRN. In a second step, the EP algorithm may learn gene representations by combining the learned annotation and identity embeddings. That is, the gene representations combine the information from the GRN structure and the annotations.

The messages may be defined by an encoding function $f_\theta$ which maps the annotations and gene identities to a vector representation. The encoding function is parameterized by $\theta$ and must be differentiable. Concretely, in an embodiment of the invention, a neural network may be used which consists of single dense layer, which amounts to an embedding lookup table. In the following, the annotations and identity of gene $g$ are referred to as $a(g)$, and the neighbors of $g$ in the GRN are referred to as $n(g)$. The notation $a(n(g))$ is used to indicate the annotations and identities of all neighbors of $g$.

Further, $h(g) = agg(f_\theta(x)|x \in a(g))$ is used to denote the current embedding of $g$, where $agg$ is an aggregation function such as taking the element-wise mean or maximum from the set of embeddings. Similarly, $\tilde{h}(g) = agg(f_\theta(x)|x \in a(n(g)))$ is used to indicate the aggregated embedding of all neighbors of $g$ in the GRN.

Mathematically, EP minimizes the following loss function:

$$\mathcal{L} = \sum_{g \in \mathrm{GRN}} \sum_{i \in \mathrm{GRN} \setminus \{g\}} \left[ \gamma + \mathrm{d}\left(\widetilde{h}(g), h(g)\right) - \mathrm{d}\left(\widetilde{h}(g), h(i)\right) \right]_+,$$

where d is the Euclidean distance, $[x]_+$ is the positive part of $x$, and $\gamma > 0$ is a margin hyperparameter.

It should be noted that, in practice, it is not feasible to evaluate the inner summation, so negative sampling may be used (for instance, as described in Kotnis, B., Nastase, V.: Analysis of the impact of negative sampling on link prediction in knowledge graphs. KnoProceedings of the 1st Workshop on Knowledge Base Construction, Reasoning and Mining (2018)) and a single random gene that is not $g$ may be evaluated. The parameters of $f_\theta$ can be updated using standard backpropagation algorithms until convergence.

For the remainder of the patient stratification pipeline 100 shown in Fig. 1, $h(g)$ is used as the embedding for the genes. It should be noted that the embeddings depend only on the GRN and annotations; thus, they can be used for multiple scRNA-seq datasets.

**Creating gene modules**

According to an embodiment of the present invention, in order to obtain the gene modules, the embeddings of the genes may be clustered using a Bayesian Gaussian Mixture Model (BGMM). For instance, the cluster centroids may be taken as summaries of the gene modules. That is, each gene module is represented by the centroid of its respective cluster. Herein, the centroid for gene module m is denoted as $C(m)$.

As a preprocessing step and for numeric stability, the embeddings may be scaled such that the values in each dimension have a mean of zero and variance of one. In the context of their analysis, the inventors found that 10 gene modules offered a good tradeoff between accuracy, computational requirements, and granularity for interpretation.

**Preparing expression data**

According to embodiments of the invention, the patient stratification pipeline 100 may include the preparation of the scRNA-seq for downstream analysis. In this context, first, a standard procedure (for instance, as described in "Integrating single-cell transcriptomic data across different conditions, technologies, and species", Nature Biotechnology 36(5), 411-420 (2018)) may be used to normalize the observed read counts per cell:

$$\widetilde{x}_{c,g} = \log_e \left[ 1 + \frac{x_{c,g}}{\sum_G x_{c,g}} \cdot 10^4 \right], \tag{1}$$

where c is a particular cell, $g$ is a gene (or transcript, etc.), $x_{c,g}$ is the observed read count of gene $g$ for cell $c$, and $x_{c,g}$ is the normalized expression value.

Then, a standard collaborative filtering approach may be used, for instance, singular value decomposition (SVD) as implemented in the Surprise package (as described in Hug, N.: Surprise: A python library for recommender systems. Journal of Open Source Software 5, 2174 (2020)), in order to account for dropout and other technical artifacts and noise. Intuitively, SVD estimates the expression of cell-gene pairs based on similar cells and genes. In particular, it solves the following optimization problem for minimizing the error between estimated and observed values:

$$\sum_{\widetilde{x}_{c,g} \in R_{train}} (\widetilde{x}_{c,g} - \hat{x}_{c,g})^2 + \lambda(b_c^2 + b_g^2 + \|q_c\|^2 + \|p_g\|^2) \tag{2}$$

$$\hat{x}_{c,g} = \mu + b_c + b_g + q_c^T p_g \tag{3}$$

where $\bar{x}_{c,g}$ and $\hat{x}_{c,g}$ are, respectively, the observed and predicted (normalized) expression values, $q_c$ and $p_g$ are their factors, and $b_c$ and $b_g$ are the biases. The set $R_{train}$ includes all non-zero values from the sc-seq data matrix. The regularizer $\lambda$ is a hyperparameter of the model, while the factors and biases are learned using an iterative stochastic gradient descent optimization algorithm.

For instance, empirically, 40 epochs may be chosen while leaving all the other parameters with their default values.

After the model parameter are learned, the expression values may be reconstructed as $\hat{x}_{c,g}$. Importantly, all expression values for all cells are updated; thus, the approach according to embodiments of the invention as disclosed herein addresses not only dropout but also other sources of noise.

As a final step, the total expression of each cell may be scaled such that the total sum is 1, as shown in (4):

$$\bar{x}_{c,g} = \frac{\hat{x}_{c,g}}{\sum_G \hat{x}_{c,g}} \tag{4}$$

As will be appreciated by those skilled in the art, it is likewise possible to employ state-of-the-art imputation methods other than the SVD approach described above. However, since the inventors found in their analysis that SVD is competitive and usually outperforms other methods, SVD was used for the remaining analysis.

**Creating cell activation vectors**

In the context of the present disclosure, an Activation Vector (AV) is the representation of a single cell by the activity level of its GMs. According to an embodiment of the invention, AVs may be created in two steps. First, an embedding may be created for each cell based on the gene embeddings learned from the GCNN. The distance between the GM centroids and the cell embedding then give the AV for that cell.

*Cell Embedding*

In an embodiment, an embedding for each cell, $h(c)$, may be obtained by linearly combining the gene embedding learned from the GCNN with the gene expression values estimated by collaborative filtering, as shown in (5):

$$h(c) = \sum^{G} \bar{x}_{c,g} \cdot h(g) \qquad (5)$$

The obtained cell embedding values may be scaled by using the same scaler applied on the gene embedding matrix before the clustering for defining the GMs.

*Cell Activation Vector*

The final cell AVs may be taken as the cosine similarity (cos) between each gene module centroid and the cell embedding:

$$av_c = [\cos(h(c), C_1), \ldots, \cos(h(c), C_M)], \qquad (6)$$

where *M* denotes the number of gene modules (e.g., *M* = 10, as outlined above).

**Stratifying patients**

The last step of the patient stratification pipeline 100 according to the embodiment of the invention illustrated in Figs. 1-4 includes training a machine learning model to predict cells which originate from responding samples and those which come from non-responding samples, for a particular drug. It is typically not possible to detect whether a single cell responds to a drug with scRNA-seq. Thus, in order to label individual cells as responders and non-responders, it may first be determined whether each sample responds to a drug or not. For example, the entire sample may be split into two portions, with one portion being treated with the drug and another portion used for single-cell sequencing. Based on the response of the portion of the sample treated with the drug, the sequenced cells can be labeled as responder or non-responder. Importantly, the sequenced cells *have not* been treated with the drug. Thus, the labels indicate whether the cells *will* respond to treatment, not whether they *have already* responded to treatment.

More specifically, the approach builds a training set based on a cohort of non-treated samples. The training set contains the AV for each cell from the samples in this cohort, and the (binary) label of each cell is whether it came from a sample which responded to treatment. A classifier may then be trained to predict whether a cell comes from a responding sample. The trained classifier may be used to classify all cells from a separated cohort of non-treated samples (used as test set) as coming from a responder or not. Each sample from this testing cohort may finally be stratified into responder or non-responder using a simple voting scheme based on the predictions of each cell from that sample.

Embodiments of the present invention use a method algorithm to represent data, obtained from high-throughput sequencing, in an Activation Vector form, that is, a representation in which each sample is expressed as the activity level of functional clusters of the genes (the GMs) contained in it.

GMs may be obtained by creating a multimodal Gene Regulatory Network, where the relations among genes are enriched with additional annotations coming from domain knowledge.

An important aspect according to embodiments of the invention lies in the method for evaluating the activity of the clusters, which is the distance between the embedding of each sample from the high-throughput data and the centroids of the GMs. This evaluation is made possible thanks to the fact that both the sample and the cluster of the genes are in the same embedding space, thus making it possible to evaluate the distance between two vectors.

It is important to recall that according to embodiments the Gene Modules are obtained from domain knowledge only. This contributes to an improvement of the explainability of the results, e.g., when the Activation Vectors are used in a Machine Learning context.

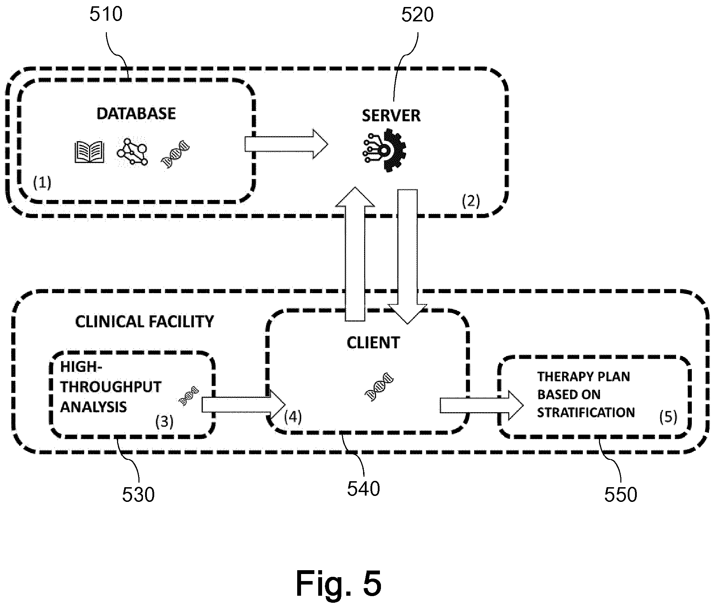

Fig. 5 illustrates an embodiment of the algorithm, along with a use case where the method for quantifying cellular activity as disclosed herein represents the core activity and where the Activation Vectors are used for patient stratification from single-cell data. Generally, patient stratification approaches are used to classify patients as responders or non-responders to specific treatments. Predicting such response *in silico* brings several advantages for both the patients and the clinicians, since the *in vivo* test of the different drug for the treatment is avoided. **In** the context of the present disclosure, it is shown how this process can be applied on a single patient, by testing the response of the high-throughput sequencing samples on different drugs.

As shown in Fig. 5, the embodiment may include a number of interacting components, namely as follows:

1. A database 510, e.g. located in the hospital, containing high-throughput expression data from patients and ad-hoc cultivated cells (cell lines), for different tumors. This database 510 may be configured to contain expression from treated and control samples, in order to have labels on the prediction. Additionally, it may be configured to contain domain knowledge acquired from different sources, like annotations and Gene Regulatory Networks.

2. A server 520, on which the algorithm is running that represents the data as Activation Vectors, and performs the stratification process through a Machine Learning algorithm. Depending on the treatment of interest, the data for that specific treatment are gathered from the database, and used for creating an Activation Vector representation of the data coming from a client 540 (see below).

3. A machine 530 that performs high-throughput data analysis from the sample obtained from a patient.

4. A client 540 that is used by the clinical facility that receives the high-throughput data from the machine 530 and sends them to the server, along with the list of drugs for which the prediction is desired.

5. A therapy plan scheduler 550 using the result of the stratification performed in the server 520. It may be configured to receive an ID of the drug to which the patient will respond, and may be used by the clinician to calculate the dosage.

Database 510

The database 510 can be located in a hospital or can be accessed through cloud services, if compliant to the privacy regulations. In an embodiment, the database 510 contains high-throughput samples of different tumor cells, treated with different drugs. For each sample, pre- and post-treatment data will be present. The samples will have multiple labels depending on the response to different drugs. The database 510 will also contain external domain knowledge, in the form of Gene-Gene interaction networks (Gene Regulatory Networks, GRN) and functional annotations of each gene. Further information about the genes, such as free text, may also be available. These data will be used to train the algorithm contained in the server 520 in order to predict the response of samples obtained from the high throughput analysis performed by machine 530 and uploaded by client 540.

A snapshot of the high-throughput data contained in database 510, where each row is a cell and each column the expression level of the genes in it, is the following. Each sample is also annotated with a label which indicates whether it came from a sample which responded to each drug. The database 510 may only store cells from pre-treatment samples.

| Cell_ID | Gene 1 | Gene 2 | Gene 3 | Gene 4 | ... | Gene 5 |
|---------|--------|--------|--------|--------|-----|--------|
| Sample 1 | 66.3 | 81.3 | 5.42 | 7.89 | ... | 6.28 |

| Cell_ID | Drug 1 | Drug 2 | Drug 3 | Drug 4 | ... | Drug 5 |
|---------|--------|--------|--------|--------|-----|--------|
| Sample 1 | Responder | Non-Responder | Responder | Non-Responder | ... | Responder |

Server 520

The server 520 contains the Activation Vector algorithm, i.e. the core method that takes as input the high-throughput data from a patient and returns the prediction on the response for drugs of interest. Specific equations and technical implementations of this algorithm may follow the principles as described in detail above.

According to an embodiment, the algorithm workflow may have an offline and an online phase, as follows: The Offline Phase may include the following steps:

Creating gene modules and cluster centroids:

    1. The domain knowledge (annotations, GRNs) related to the genes of interest are merged together thus creating a multimodal graph.
    2. The nodes of this graph are embedded and clustered in GMs.
    3. The cluster centroids, cluster assignments, and gene embeddings are saved.

Training drug response prediction models:

    1. AVs are constructed from the high throughput sample dataset in database 510.

        a. As an optional step, the dropout values of the high-throughput data on the database may be imputed through a Collaboration Filtering algorithm.
        b. All rows (cells) of the data values are rescaled so the sum over all the genes is 1. The rescaled data and the embedded genes nodes are linearly combined, and an embedding for each cell is obtained.

    c. The distance between each cell and each GM centroid is evaluated. This results in having each cell represented as an Activation Vector.

    2. An ML classifier is trained to predict the label of each cell.

    3. The processes 1-2 are repeated for each drug of interest.

The Online Phase may include the following steps (wherein the data for this online phase may come from the high-throughput data acquisition performed by machine 530 that is provided via the client components 540):

    1. As an optional step, the dropout values of the high-throughput data on the database may be imputed through a Collaboration Filtering algorithm.

    2. Each row (sample) of the data values may be rescaled so the sum over all the genes is 1. The rescaled data and the embedded genes nodes are linearly combined and an embedding for each sample is obtained.

    3. The distance between each cell and each GM centroid is evaluated. This results in having each sample represented as an Activation Vector.

    4. The appropriate trained ML is used to predict a label (responder/non-responder) for each sample.

    5. A voting process may establish the total response of a patient to that specific drug with a confidence score. Specifically, the fraction of samples (cells) predicted to respond give the confidence that the patient will respond to the drug. Also, according to an embodiment it may be provided that the most important Activation Vectors used in the prediction are returned, in order to give an additional explanation for the results to the clinicians.

The prediction of the drug that guarantees a response is returned to the client 540 and passed to the clinician in the therapy scheduler 550.

High-throughput data acquisition 530

This can be any machine that takes in input a sample from a patient and performs the high-throughput sequencing (e.g. single-cell sequencing).

Client 540

The client 540 may be used by a clinician to upload the sequencing data to the server 520 in order to perform the *in silico* prediction on the response. The server 520 may be configured to return the response prediction to the client 540, who may be configured to forward the response to the therapy scheduler 550. The clinician may then use the therapy scheduler 550 to visualize the selected treatment.

Treatment scheduler 550

The scheduler 550 may be implemented as a front-end application that a clinician may use to load the prediction outcome, and start scheduling the therapy using the selected treatment. In particular, the scheduler 550 may be configured to automatically create an appropriate prescription for the patient and, possibly, to forward it to a pharmacy.

According to a slightly different embodiment, the present invention provides a computer-implemented method and a processing system for quantifying cellular activity from high throughput sequencing data where the Activation Vectors are used for patient stratification from bulk sequencing data.

While the approach of the embodiment described above is based on single-cell sequencing data, in which each biological sample results in many observations (one for each cell), the approach is also applicable for bulk sequencing, in which each biological sample results in a single observation (a kind of average over all cells in the sample). The GM and AV approach can also be used to create sample embeddings for bulk sequencing data. The only required change is to Step 5 of the online phase (see above) performed by the server 520 (i.e., the "voting" step). Since each bulk sequencing sample consists of only a single observation, there is no opportunity to vote. Instead, the server 520 may be configured to directly use the prediction from the trained ML model in Step 4 of the online phase (see above) to calculate the total response of a patient to that specific drug with a confidence score.

As outlined already above, currently, standard sequencing analysis, such as bulk RNA sequencing, allows to obtain a single reading for the various transcripts/gene in one patient. All of the prior art approaches discussed above base their stratification on the expression of single genes (or proteins, metabolites), so a large number of patients are needed for making these model efficient. In other words, in order to perform any kind of ML-based prediction (such as patient stratification) it is necessary to have a large availability of patients, which is rarely possible. Embodiments of the present invention, on the contrary, rely instead on a small number of patients, since high-throughput, single-cell sequencing data allows to obtain a large amount of samples per patient. In this case for a single patient, cell readings in the order of the thousand can be obtained. This enables the possibility to perform predictions with few amount of samples.

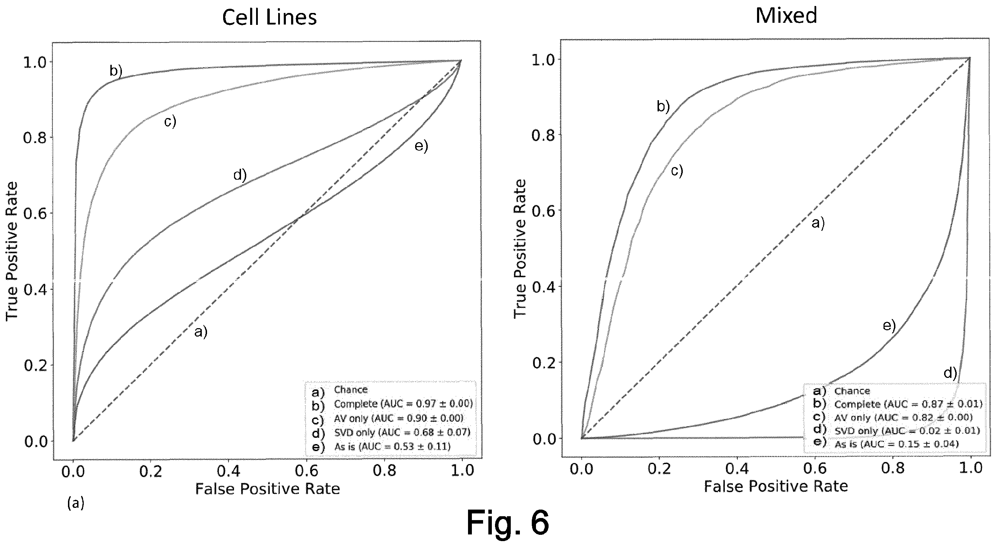

Furthermore, embodiments of the present invention create a representation of data that looks at the genetic signature (Gene Modules), opposed to the standard techniques that look at the differential activity of the single genes. This leads to more accurate predictions, as they can be obtained from Fig. 6, which illustrates the AUC (Area Under the Curve) on the prediction of the response of the cells in a patient stratification process on sc-seq data. When the AV based method according to the invention is not used (see curves a) 'chance', d) 'SVD (singular value decomposition) only' and e) 'as is'), thus relying only on the expression values from the single gene, the results are quite mediocre compared to curve c) 'AV only' and curve d) 'complete', i.e. SVD + AV. Generally, the approach according to embodiments of the invention makes ML-based predictions explainable, due to the fact that the predicted response in the cell, can be decomposed to the single Activation Vector contribution, which has a real-world meaning, thanks to the domain knowledge used during its construction.

According to an application scenario, embodiments of the present invention focus on the stratification of patients affected by Multiple Myeloma (MM). MM is one of the most common forms of hematopoietic malignancy, characterized by clonal expansion of malignant B-cell-derived plasma cells in the bone marrow (cf. Rajkumar, S.V.: Multiple myeloma: 2012 update on diagnosis, risk-stratification, and management. American Journal of Hematology 87, 77-88 (2012)). Currently, proteasome inhibitors (PIs) are the most effective therapies for MM: among the most important ones, bortezomib (Velcade) was the first PI approved for treatment of MM (in 2003), followed by carfilzomib (Kyprolis) (approved in 2012), and oral PIs include ixazomib (Ninlaro, 2015), and panobinostat (Farydak). In this context, experiments have been carried out that perform a stratification of patients treated with panobinostat, by looking at their genetic signatures derived from single-cell sequencing techniques.

For this work, scRNA-seq expression data were used from 4 Multiple Myeloma patients (samples were collected at BioQuant, Heidelberg, DE), treated with panobinostat. Primary cells from patient samples were sorted by CD138 to isolate B cells. These cells were then treated with either a control (DMSO) or panobinostat, and finally underwent 5'-end scRNA-seq. In total of 29 997 cells and 26 063 different transcripts were quantified from the patient samples. Additionally, 3 cell line models for MM were also treated with either DMSO or panobinostat and then sequence. A total of 6 478 cells and 11 723 transcripts were quantified from the cell lines.

Among the patient samples, domain experts qualitatively determined that three samples responded to the drug, while one sample did not. Among the cell lines, two responded to the drug and one did not. The samples treated with DMSO were considered as indicative of pre-treatment samples, and those form the basis of the patient stratification algorithms as described in the present disclosure. The samples treated with panobinostat were not used other than for determining the labels for the pretreatment cells. Table 1 describes in detail the 7 samples used in this study.

Table 1: Dataset description

| Sample | Origin | Response | #cells |
|--------|--------|----------|--------|
| 01 | Patient | Responder | 10928 |
| 02 | Patient | Non-Responder | 5996 |
| 03 | Patient | Responder | 5551 |
| 04 | Patient | Responder | 7522 |
| 05 | Cell Line | Non-Responder | 1485 |
| 06 | Cell Line | Responder | 2503 |
| 07 | Cell Line | Responder | 2490 |

As external knowledge, a B cell-specific gene regulatory network was used to obtain information on the interactions among genes. Gene Ontology (GO) (cf. Generic GO Slim Subset. http://current.geneontology.org/ontology/subsets/goslim_generic.obo) annotations were collected for 18 177 genes. Those genes for which no GO annotations were available were removed from the GRN.

As the gene set used for analysis, the top-2 000 most-expressed genes were kept, based on the number of cells in which the gene is detected. Then, all genes which were not included in the GRN were removed. In total, the final network and analysis was based on 1 713 genes. Table 2 provides summary information of the connectivity of GRN and GO annotations.

Table 2: Summary of the B-Cell GRN properties

| | Nodes | Edges | Average degree |
|---|---|---|---|
| B_Cell-GRN | 1713 | 10089 | 11.78±16.44 |

## Results

*Gene embeddings and gene modules*

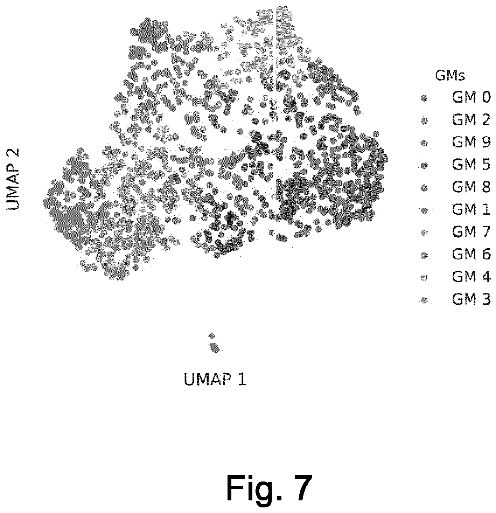

We first qualitatively evaluated the gene embeddings and the resulting gene modules (GMs). The two-dimensional UMAP (cf. McInnes, L., Healy, J., Melville, J.: UMAP: Uniform Manifold Approximation and Projection for Dimension Reduction (2018). arXiv:1802.03426 [stat.ML]) projection of the embeddings are shown in Fig. 7. The clustering of the latent representation of the genes is based on the gene regulatory network and Gene Ontology annotations. Each cluster is a gene module.

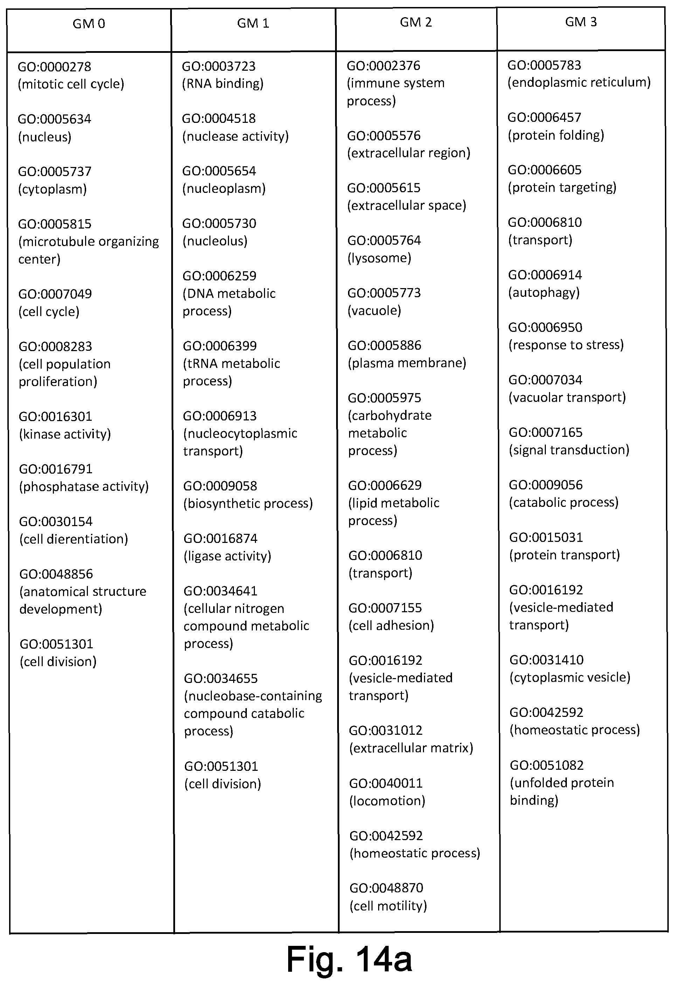

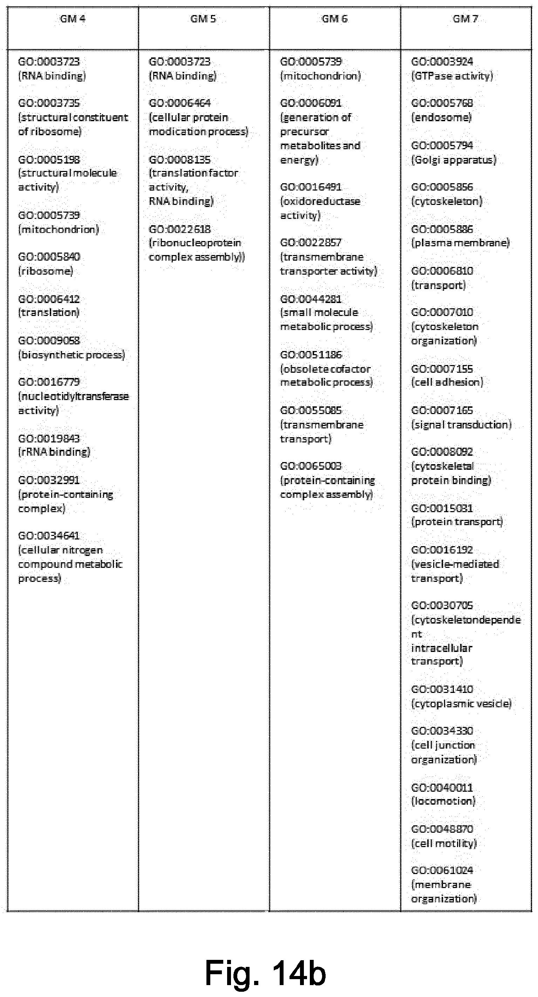

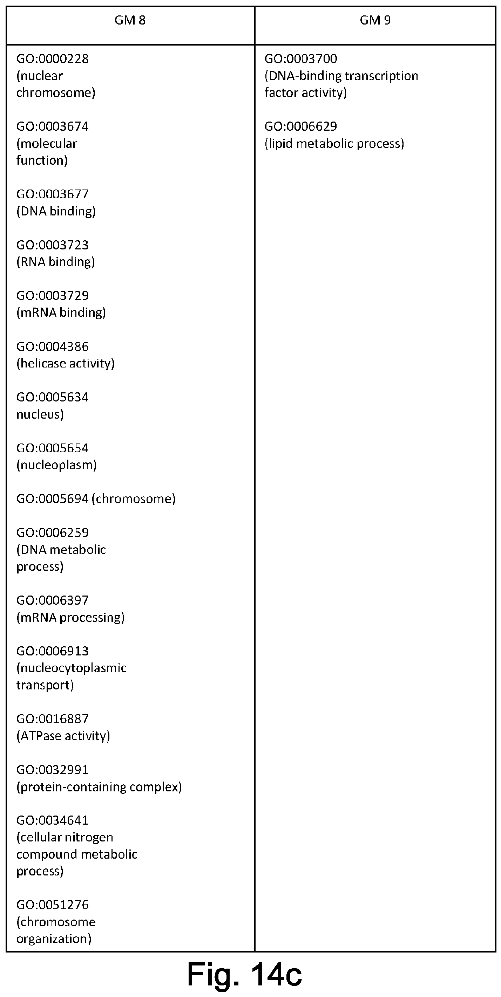

Further, we performed enrichment tests (hypergeometric test with Bonferroni correction, $p < 0.05$ was taken as significant) in order to detect overrepresented Gene Ontology (GO) annotations in each GM. The GOs contained in the clusters is shown in the table depicted in Fig. 14 a-c. In particular, we only considered the GO Slim (cf. Generic GO Slim Subset. http://current.geneontology.org/ ontology/subsets/goslim_generic.obo) set of annotations.

Importantly, by manually inspecting the enriched GO terms for each GM, we see that they form biologically meaningful groups. For example, GM 4 represents proteosomal activity, while GM1 could be categorized as nuclear activity. This analysis supports our hypothesis that the GMs meaningfully combine the structured domain knowledge from the GRN as well as the annotations into a single, coherent representation.

*Imputation*

We next validated the performance of our collaborative filtering-based imputation approach. In particular, we compared our proposed approach with several state-of-the-art imputation approaches (MAGIC (cf. D. van Dijk et al.: Recovering gene interactions from single-cell data using data diffusion. Cell 174(3), 716-72927, 2018), SAVER (cf. M. Huang et al.: SAVER: gene expression recovery for single-cell RNA sequencing. Nature Methods 15, 539-542, 2018), scImpute (cf. W.V. Li et al.: An accurate and robust imputation method scImpute for single-cell RNA-seq data. Nature Communications 9(997), 2018). For comparison, we used the GSE45719 dataset (mouse pre-implantation embryos at 10 different development stages), cf. Series GSE45719. https://www.ncbi.nlm.nih.gov/geo/query/ acc.cgi?acc=GSE45719. As the evaluation metric, we used the Pearson correlation coefficient (PCC).

In order to analyze the accuracy of the SVD reconstruction as a function of the amount of dropout (that is, missing values) in the gene expression matrix, we performed a sensitivity test. In particular, we downsampled the number of observed genes for each cell. The process was repeated with different observation rates. The model performance was evaluated using the Root Mean Squared Error (RMSE) metric. The workflow consisted of 5 steps.

1. Splitting the cell-gene pairs into train, validation and test sets
2. Setting the observation rate (20%, 40%, 60%, 80%, 100%) of the average number of expressed genes in each cell (in the training set)
3. For each cell in the training set, downsampling the number of observed genes according to the observation rate. The downsampling in each cell was performed by picking the number of observed genes for that cell from a standard normal distribution with mean equal to the observation rate and standard deviation equal to 10. We then dropped out all other expression values from that cell.
4. Optimizing the hyperparameters of the SVD model using a grid search and the validation set.
5. Lastly, the model is used on the test set and the RMSE is again calculated like in the previous step.

For each observation rate, we repeated the analysis five times to assess the variance in performance due to randomness. The validation and test sets were kept the same across all observation rates and samples to ensure the results are comparable for all settings. Even if RMSE is evaluated only for the known entities, having very low values for the error makes highly probable that the reconstructed dropouts will be meaningful.

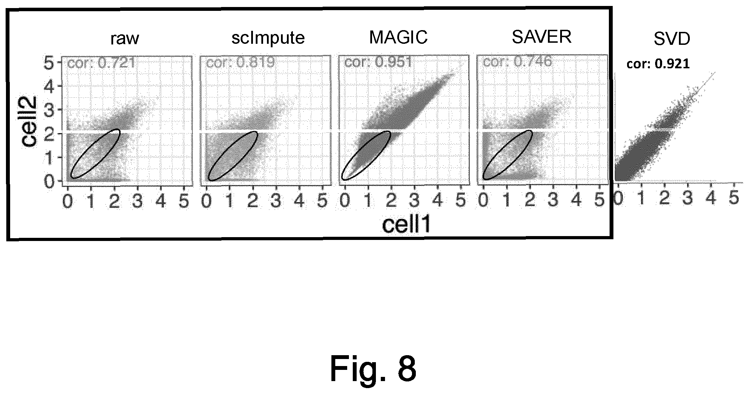

Fig. 8 illustrates imputation benchmarking based on a comparison of the imputed values of two replicate cells. Since the cells are from the same stage of development, it is expected that the expression values of most gene should be very similar. Thus, we would expect most imputed values to fall along the main diagonal. As can be obtained from Fig. 8, our collaborative filtering-based imputation is competitive with the state of the art, PCC: 0.92 vs 0.75 (SAVER), 0.82 (scImpute), 0.95 (MAGIC). More specifically, our SVD approach results in a higher PCC among the imputed values between the two cells than most of the other methods.

*Activation vectors*

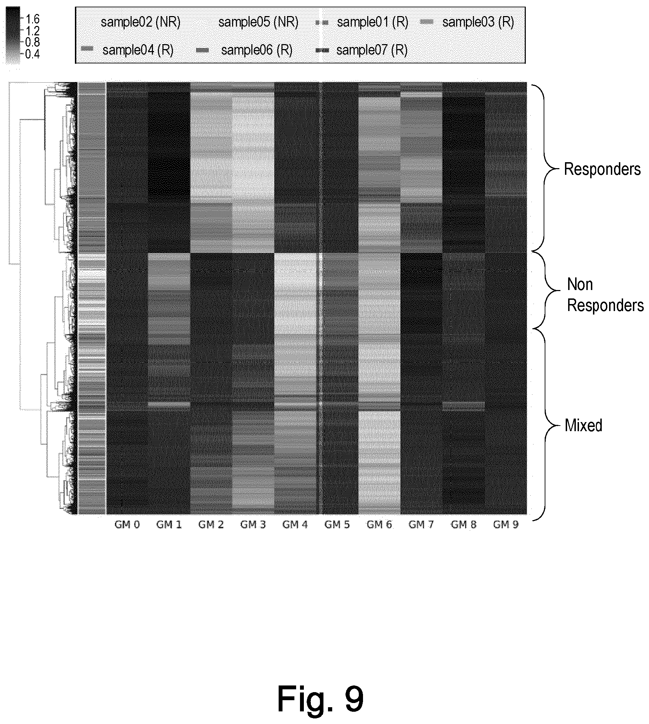

As a first qualitative result, we performed a hierarchical clustering of the AVs for cells from all the non-treated samples. Fig. 9 depicts an hierarchical clustering of activation vectors for all samples treated with the control. Cells from the non-responder samples are labeled in a light color. As can be obtained from Fig. 9, the cells tend to form clusters of responders and non-responders. This suggests that a machine learning algorithm may succeed in predicting responders and non-responders. On the other hand, the cells from individual samples do not all cluster together. Encouragingly, this suggests that the AVs capture trends across samples rather than simply reflecting sample-specific artifacts.

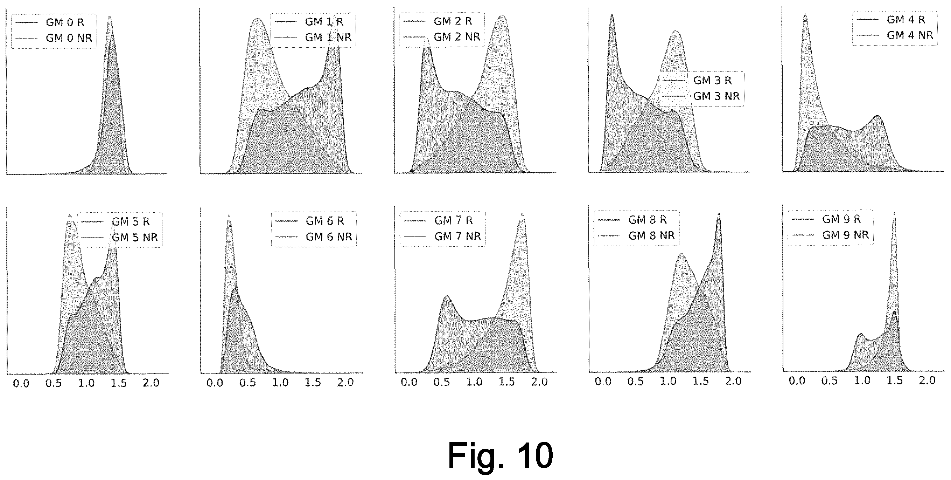

We next qualitatively investigated the distribution of values for responders and non-responders of the pre-treatment samples for the AVs. As shown in Fig. 10, several of the GMs have highly discriminative distributions between responders (R) and non-responders (NR). This again suggests that the GMs capture meaningful biological activity and that a classifier may distinguish responders and non-responders.

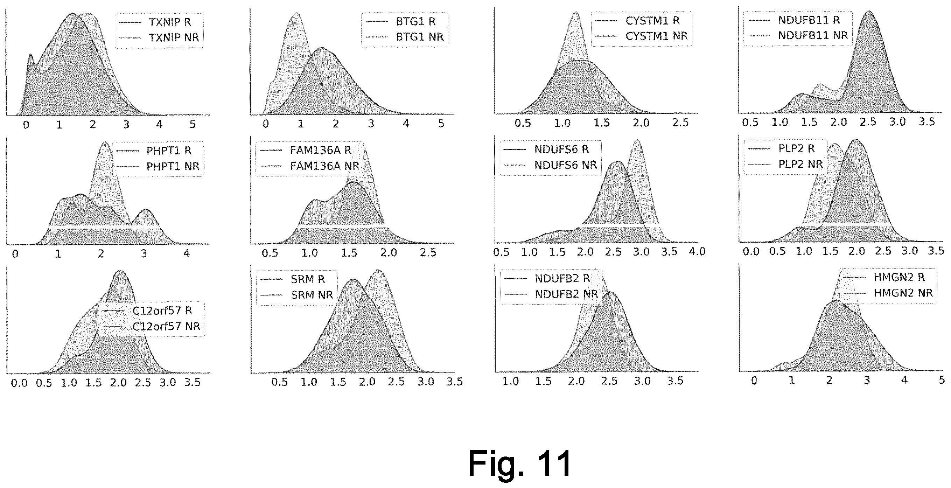

For comparison, we identified differentially expressed genes between the responder and non-responder samples using standard approaches. Fig. 11 shows gene expression distributions for top-ranked genes, i.e. the kernel density estimation of the distributions of expression values for the most important genes used by the classifier. The distributions are very similar, i.e. not discriminative, among the responders (R) and non-responders (NR), thus showing that using single gene expression values as discriminative features may not lead to good predictions. This highlights the advantage of using GMs for this classification task.

*Machine learning evaluation*

We next quantitatively evaluated the Activation Vectors (AVs) for patient stratification. For evaluation, we split the patient and cell line samples into training and testing cohorts.

We evaluated our approach using two different training scenarios, based on how we split the samples from the patients and the cell lines. In the first set of experiments ("Mixed setup" in the results), both cell line and patient samples were used in both the training and testing datasets. In the second ("CL setup"), only cell line samples were used for training while patient samples were used for testing. In both cases, cells from each sample appeared in only the training or only the test set. This ensures the machine learning model does not simply memorize what cells from a particular sample look like.

For both scenarios, we considered four unique computational pipelines: one in which both the AV representation and the imputation were used ("complete"), one with only the AVs but not the imputation ("AV only"), one with only the imputation and not the AVs ("SVD only"), and the last one without both ("as is"). For all evaluations, we used 10-fold cross validation to ensure appropriate error estimates are available.

The selected Machine Learning classifier for this task was a Logistic Regression (LR). We set two hyperparameters (tolerance for early stopping to $10e-3$, maximum number of iterations to 1000). For the class weight (unweighted or balanced), coefficient penalty ($\ell-1$ or $\ell-2$), and inverse of regularization strength ($\{1, 10, 100, 1000\}$), we selected the best values using a grid search with crossvalidation.

While splitting the dataset in train and test sets as described above, we ensured that at least one non-responder sample and one responder sample was in both splits. As described above, in some cases, SVD was not used to impute the missing gene values. In those cases, missing values were imputed with the average expression of that gene across all cells for which it was observed.

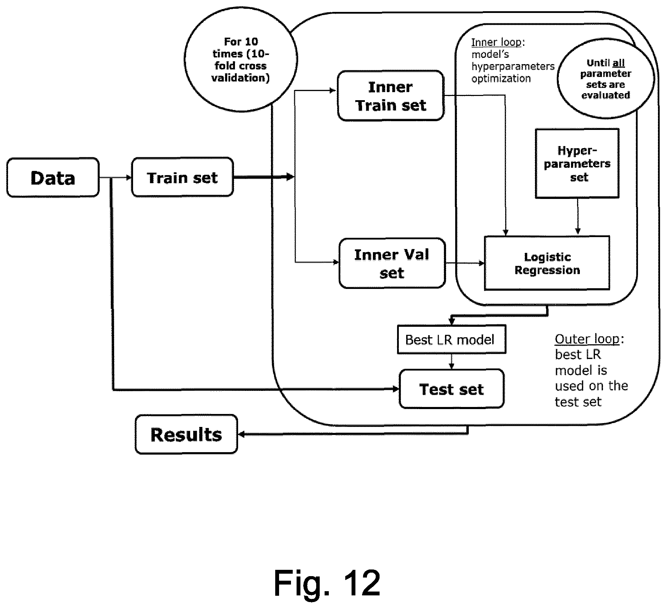

Fig. 12 shows how each experiment was performed: the train set was split in 10 folds by using the StratifiedKFold function in sklearn (which ensures that the class proportion of the dataset are preserved in the single fold). For each training set split, an inner training set and an inner validation set were created by further splitting the training set. The LR hyperparameters were then selected by training on the inner training set and evaluating on the inner validation set. The hyperparameters for which the LR returned the best Area under the ROC curve (AuROC) value on the inner validation set was selected as the best model for that training fold. We used the selected model to make predictions for the test set, and we obtained the AuROC value for that fold. The experiment was repeated for the remaining 9 folds, and in the end we evaluated the average AUC among the 10 folds.

*Cell classification*

Fig. 6 shows the results both for the "CL setup" (left) and for the "Mixed setup" (right). More specifically, Fig. 6 show the area under the receiver operating characteristic curve (AuROC) of each of the computations pipelines for both training scenarios. These results clearly demonstrate that the use of the activation vectors significantly improves the classification performance compared to using baseline methods, with or without imputed expression values. In both training scenarios, combining the activation vectors with imputation led to the best results.

For the "Mixed setup", Fig. 6 shows that "SVD only" and "as is" performed especially poorly (AuROC < 0.5). In these cases, the trained model predicts a very high probability (> 0.95) that nearly all of the cells in the testing samples come from responders; the cells from non-responders are consistently predicted to have an even higher probability (> 0.99) of coming from a responder. This phenomenon results in the very low AuROC values.

*Patient stratification*

Table 3 shows the quantitative results of the patient stratification predictions.

Table 3: Accordance probability of Sample response

|  | Test Set | Real | Prediction AV+SVD | Prediction AV | Prediction SVD | Prediction (as is) |
|---|---|---|---|---|---|---|
| Mixed setup | Sample 04 | R | 98.4% | 96.5% | 58.9% | 95.2% |
|  | Sample 05 | NR | 62.6% | 64.6% | 99.9% | 100.0% |
| Cell Lines setup | Sample 01 | R | **93.6%** | 78.9% | 80.1% | 55.5% |
|  | Sample 02 | NR | **4.1%** | 7.6% | 25.4% | 58.7% |
|  | Sample 03 | R | **62.9%** | 21.1% | 6.1% | 22.6% |
|  | Sample 04 | R | **94.5%** | 74.5% | 48.9% | 91.7% |

The percentage shows the probability that the sample is predicted as responder. The actual label is shown in the second column. The Cell Lines setup is shown to be more robust at predicting the right behavior of the sample. For both of the dataset construction scenarios, our proposed approach (collaborative filtering-based imputation and the use of AVs which encode both domain knowledge and expression data) outperforms the other baselines. In both scenarios, the AVs significantly improve the results compared to directly using the gene expression values. While we cannot draw strong conclusions due to the low number of available samples, this provides evidence of the value of including domain knowledge for stratifying patients.

*Gene Ontology and model interpretation*

The pipeline presented in accordance with embodiments of the present invention is interpretable by design, since all the predictions are built on a set of features (the GMs) that can be mapped to specific functions. In particular, as described earlier, each GM is associated with a set of GO annotations. Further, the importance of each GM in the classifier is taken as the absolute value of its corresponding coefficient in the logistic regression model. Similar "feature importance" approaches (cf. M.T. Ribeiro et al.: "why should i trust you?": Explaining the predictions of any classifier. In: Proceedings of the 22nd ACM SIGKDD Conference on Knowledge Discovery and Data Mining, 2016) could be used for other machine learning models, as well.

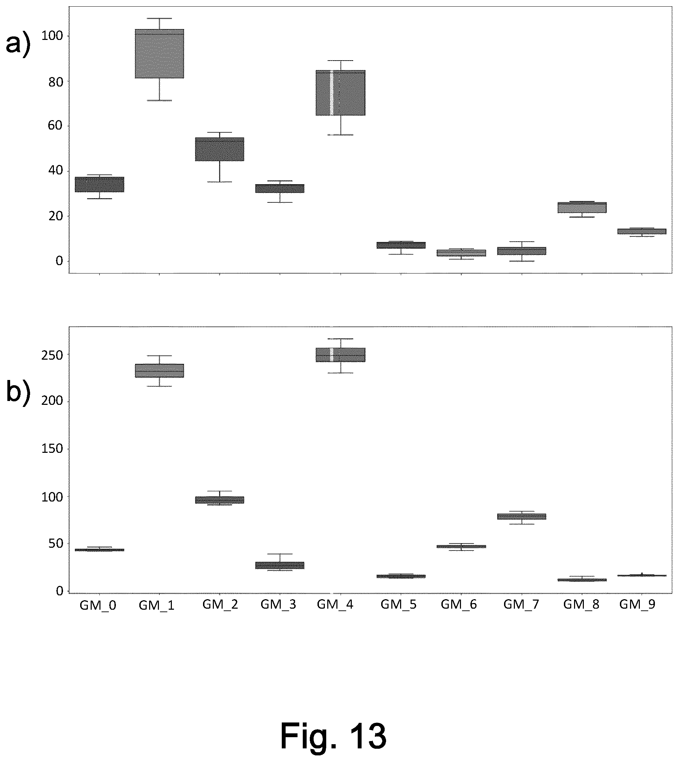

Fig. 13 shows the distribution of importances of each GM across all splits of the cross-validation. Specifically, Fig. 3 depicts boxplots for the feature importance for each of the 10 gene modules (GMs) for each of the 10 folds, for the (a) Cell Lines and (b) Mixed setup. The feature importance is taken as the absolute value of the coefficient for the respective GM in the trained logistic regression model.

Fig. 13 shows that GM #4 is consistently an important, i.e. discriminative, feature for the classifier. GM #4 is highly enriched in proteasomes, and this is in line with the work by Tanaka et al. (cf. Tanaka, K.: The proteasome: Overview of structure and functions. Proceedings of the Japan Academy, Series B 85, 12-36, 2009), in which it is shown that panobinostat plays an important role in downregulating proteasomal activity. Indeed, Fig. 10 shows that non-responders already have low activity for GM #4; consequently, panobinostat is not able to help these patients because there is already low proteasomal activity. On the other hand, responders have a moderate to high activity level for GM #4. In these cases, then, panobinostat can effectively downregulate proteasomal activity and lead to response in the patients.

To summarize, in accordance with embodiments of the present invention, a pipeline for patient stratification has been developed, which uses the activation level of functional gene groups in the cells. This can be obtained by combining the data coming from single-cell RNA sequencing (scRNA-seq) with external prior knowledge, e.g. by means of a graph convolutional neural network. A key component of embodiments of the invention is the creation of Gene Modules (GMs) based on the external prior knowledge. By focusing on the activation of GMs within cells to predict their response to treatment, the analysis according to embodiments of the invention is robust against noise from individual genes. In addition, embodiments of the invention propose a simple collaborative filtering-based approach to account for technical and biological noise in the scRNA-seq data, further improving the resilience of the approach. Empirically, it could be demonstrated on a set of multiple myeloma patient and cell line samples that GM-based predictions led to an AuROC performance of 0.97, while directly using the individual gene expression values only lead to 0.55, hardly better than guessing.

Deeper analysis showed that the GM-based approach according to embodiments of the invention is able to directly reveal relevant biological functions; this is in contrast to classical differential expression analysis, which only identifies individual genes. For example, in the context of the present invention, it has been shown that the GM associated with proteasomal activity was quite important for predicting response to panobinostat. This is in agreement with existing literature on panobinostat.

**Claims**

1. A computer-implemented method for patient stratification using high throughput RNA sequencing data, wherein the sequencing data include single-cell RNA sequencing data obtained by performing single-cell RNA sequencing, scRNA-seq, of samples taken from a patient, the method comprising:

    using a gene regulatory network, GRN, and gene annotations coming from domain knowledge to generate a multimodal knowledge graph, wherein the nodes of the multimodal knowledge graph are genes and wherein the gene annotations enrich the relations among the genes, wherein the multimodal knowledge graph is generated by combining the GRN with the gene annotations into single vector representations of the genes of the GRN, which are embeddings of the genes of the GRN;
    creating a number of gene modules, GMs, by clustering the embeddings of the genes of the GRN;
    embedding the samples of the sequencing data into the multimodal knowledge graph;
    generating, for each sample of the sequencing data, an activation vector in which the respective sample is expressed as the distances between the embedding and the centroids of each of the number of GMs;
    using the activation vectors as input for training a machine learning algorithm to predict a response of individual cells to a drug of interest;
    using the trained machine learning algorithm to predict a label 'responder' or 'non-responder' for each cell of the sequencing data; and
    establishing by a voting process the total response of the patient to said drug of interest with a confidence score, wherein the fraction of cells predicted to respond to said drug represents the confidence that the patient will respond to said drug.

2. The method according to claim 1, wherein embedding the samples of the sequencing data into the multimodal knowledge graph includes linearly combining the sequencing data with the embeddings of the genes.

3. The method according to claim 1 or 2, further comprising:

    using a graph convolutional neural network, GCNN, to create the embeddings of each gene based on the GRN with the gene annotations; and
    clustering the embeddings and providing the clusters as the GMs.

4. The method according to any of claims 1 to 3, further comprising, prior to generating the activation vectors: applying a collaboration filtering algorithm to remove dropout values and other sources of noise from the high throughput sequencing data.

5. The method according to any of claims 1 to 4, further comprising: providing as output one or more of the most important activation vectors used in the prediction as an explanation for the obtained results.

6. A processing system for patient stratification using high throughput RNA sequencing data, in particular for executing a method according to anyone of claims 1 to 5, wherein the sequencing data include single-cell RNA sequencing data obtained by performing single-cell RNA sequencing, scRNA-seq, of samples taken from a patient, the system comprising one or more processors configured to

    use a gene regulatory network, GRN, and gene annotations coming from domain knowledge to generate a multimodal knowledge graph, wherein the nodes of the multimodal knowledge graph are genes and wherein the gene annotations enrich the relations among the genes, wherein the multimodal knowledge graph is generated by combining the GRN with the gene annotations into single vector representations of the genes of the GRN, which are embeddings of the genes of the GRN;

create a number of gene modules, GMs, by clustering the embeddings of the genes of the GRN;

embed the samples of the sequencing data into the multimodal knowledge graph;

generate, for each sample of the sequencing data, an activation vector in which the respective sample is expressed as the distances between the embedding and the centroids of each of the number of GMs;

use the activation vectors as input for training a machine learning algorithm to predict a response of individual cells to a drug of interest;

use the trained machine learning algorithm to predict a label 'responder' or 'non-responder' for each cell of the sequencing data; and

establish by a voting process the total response of the patient to said drug of interest with a confidence score, wherein the fraction of cells predicted to respond to said drug represents the confidence that the patient will respond to said drug.

7. The processing system according to claim 6, further comprising a database (510) containing high-throughput samples of different tumor cells, treated with different drugs, together with the multimodal knowledge graph.

8. The processing system according to claim 7, further comprising a server (520) configured to use multimodal knowledge graph from the database (510) for training a machine learning algorithm based on the activation vectors to predict a response of individual cells to an applied drug.

9. The processing system according to claim 8, further comprising a client (540) configured to be used by a clinician to upload high throughput sequencing data obtained from a patient to the server (520), wherein the server (520) is configured to

use the trained machine learning algorithm to predict a label 'responder' or 'non-responder' for each cell of the sequencing data, and

return the prediction outcome including the drug(s) that yield(s) a response to the client (540).

10. The processing system according to claim 9, further comprising a treatment scheduler (550) as a front-end application configured to be used by a clinician to load the prediction outcome and to select a treatment.

11. A non-transitory computer-readable medium comprising code for causing one or more processors of a processing system to:

use a gene regulatory network, GRN, and gene annotations coming from domain knowledge to generate a multimodal knowledge graph, wherein the nodes of the multimodal knowledge graph are genes and wherein the gene annotations enrich the relations among the genes, wherein the multimodal knowledge graph is generated by combining the GRN with the gene annotations into single vector representations of the genes of the GRN, which are embeddings of the genes of the GRN;

create a number of gene modules, GMs, by clustering the embeddings of the genes of the GRN;

embed samples of high throughput sequencing data into the multimodal knowledge graph, wherein the sequencing data include single-cell RNA sequencing data obtained by performing single-cell RNA sequencing, scRNA-seq, of samples taken from a patient;

generate, for each sample of the sequencing data, an activation vector in which the respective sample is expressed as the distances between the embedding and the centroids of each of the number of GMs;

use the activation vectors as input for training a machine learning algorithm to predict a response of individual cells to a drug of interest;

use the trained machine learning algorithm to predict a label 'responder' or 'non-responder' for each cell of the sequencing data; and

establish by a voting process the total response of the patient to said drug of interest with a confidence score, wherein the fraction of cells predicted to respond to said drug represents the confidence that the patient will respond to said drug.

**Patentansprüche**

1. Computerimplementiertes Verfahren zur Patientenstratifizierung unter Verwendung von Hochdurchsatz-RNA-Sequenzierungsdaten, wobei die Sequenzierungsdaten Einzelzell-RNA-Sequenzierungsdaten umfassen, die durch Durchführung einer Einzelzell-RNA-Sequenzierung, scRNA-seq, an von einem Patienten entnommenen Proben erhalten werden, wobei das Verfahren umfasst:

Verwenden eines Genregulationsnetzwerks, GRN, und von aus domänenspezifischem Wissen stammenden Genannotationen zum Erzeugen eines multimodalen Wissensgraphen, wobei die Knoten des multimodalen Wissensgraphen Gene sind und wobei die Genannotationen die Beziehungen zwischen den Genen anreichern, wobei der multimodale Wissensgraph durch Kombinieren des GRN mit den Genannotationen zu einzelnen Vektordarstellungen der Gene des GRN erzeugt wird, welche Einbettungen der Gene des GRN darstellen;
Erzeugen einer Anzahl von Genmodulen, GMs, durch Clustern der Einbettungen der Gene des GRN;
Einbetten der Proben der Sequenzierungsdaten in den multimodalen Wissensgraphen;
Erzeugen eines Aktivierungsvektors für jede Probe der Sequenzierungsdaten, wobei die jeweilige Probe als die Abstände zwischen der Einbettung und den Schwerpunkten jedes der Anzahl von GMs ausgedrückt wird;
Verwenden der Aktivierungsvektoren als Eingabe zum Trainieren eines maschinellen Lernalgorithmus zur Vorhersage einer Reaktion einzelner Zellen auf ein interessierendes Arzneimittel;
Verwenden des trainierten maschinellen Lernalgorithmus zur Vorhersage eines Labels "Responder" oder "Non-Responder" für jede Zelle der Sequenzierungsdaten; und
Bestimmen der Gesamtreaktion des Patienten auf das betreffende Arzneimittel mittels eines Abstimmungsverfahrens mit einem Konfidenzwert, wobei der Anteil der Zellen, die als auf das Arzneimittel reagierend vorhergesagt werden, die Konfidenz darstellt, dass der Patient auf das Arzneimittel ansprechen wird.

2. Verfahren nach Anspruch 1, wobei das Einbetten der Proben der Sequenzierungsdaten in den multimodalen Wissensgraphen ein lineares Kombinieren der Sequenzierungsdaten mit den Einbettungen der Gene umfasst.

3. Verfahren nach einem der Ansprüche 1 oder 2, ferner umfassend:

Verwenden eines graphenbasierten Faltungs-Neuronalen Netzwerks (Graph Convolutional Neural Network), GCNN, zum Erzeugen der Einbettungen jedes Gens basierend auf dem GRN mit den Genannotationen; und
Clustern der Einbettungen und Bereitstellen der Cluster als Genmodule, GMs.

4. Verfahren nach einem der Ansprüche 1 bis 3, ferner umfassend, vor dem Erzeugen der Aktivierungsvektoren:
Anwenden eines Kollaborationsfilterungs-Algorithmus (collaboration filtering algorithm), um Dropout-Werte und andere Rauschquellen aus den Hochdurchsatz-Sequenzierungsdaten zu entfernen.

5. Verfahren nach einem der Ansprüche 1 bis 4, ferner umfassend:
Bereitstellen als Ausgabe eines oder mehrerer der wichtigsten bei der Vorhersage verwendeten Aktivierungsvektoren als Erklärung für die erhaltenen Ergebnisse.

6. Verarbeitungssystem zur Patientenstratifizierung unter Verwendung von Hochdurchsatz-RNA-Sequenzierungsdaten, insbesondere zur Ausführung eines Verfahrens nach einem der Ansprüche 1 bis 5, wobei die Sequenzierungsdaten Einzelzell-RNA-Sequenzierungsdaten umfassen, die durch Durchführung einer Einzelzell-RNA-Sequenzierung, scRNA-seq, an von einem Patienten entnommenen Proben erhalten werden, wobei das System ein oder mehrere Prozessoren umfasst, die dazu konfiguriert sind:

ein Genregulationsnetzwerk, GRN, und aus domänenspezifischem Wissen stammende Genannotationen zum Erzeugen eines multimodalen Wissensgraphen zu verwenden, wobei die Knoten des multimodalen Wissensgraphen Gene sind und wobei die Genannotationen die Beziehungen zwischen den Genen anreichern, wobei der multimodale Wissensgraph durch Kombinieren des GRN mit den Genannotationen zu einzelnen Vektordarstellungen der Gene des GRN erzeugt wird, welche Einbettungen der Gene des GRN darstellen;
eine Anzahl von Genmodulen, GMs, durch Clustern der Einbettungen der Gene des GRN zu erzeugen;
die Proben der Sequenzierungsdaten in den multimodalen Wissensgraphen einzubetten;
für jede Probe der Sequenzierungsdaten einen Aktivierungsvektor zu erzeugen, wobei die jeweilige Probe als die Abstände zwischen der Einbettung und den Schwerpunkten jedes der Anzahl der GMs ausgedrückt wird;
die Aktivierungsvektoren als Eingabe zum Trainieren eines maschinellen Lernalgorithmus zur Vorhersage einer Reaktion einzelner Zellen auf ein interessierendes Arzneimittel zu verwenden;
den trainierten maschinellen Lernalgorithmus zu verwenden, um für jede Zelle der Sequenzierungsdaten ein Label "Responder" oder "Non-Responder" vorherzusagen; und
mittels eines Abstimmungsverfahrens die Gesamtreaktion des Patienten auf das betreffende Arzneimittel mit einem Konfidenzwert zu bestimmen, wobei der Anteil der als reagierend vorhergesagten Zellen die Konfidenz darstellt, dass der Patient auf das Arzneimittel ansprechen wird.

**7.** Verarbeitungssystem nach Anspruch 6, ferner umfassend eine Datenbank (510), die Hochdurchsatzproben verschiedener Tumorzellen, die mit unterschiedlichen Arzneimitteln behandelt wurden, zusammen mit dem multimodalen Wissensgraphen enthält.

**8.** Verarbeitungssystem nach Anspruch 7, ferner umfassend einen Server (520), der dazu konfiguriert ist, den multimodalen Wissensgraphen aus der Datenbank (510) zum Trainieren eines maschinellen Lernalgorithmus basierend auf den Aktivierungsvektoren zu verwenden, um eine Reaktion einzelner Zellen auf ein appliziertes Arzneimittel vorherzusagen.

**9.** Verarbeitungssystem nach Anspruch 8, ferner umfassend einen Client (540), der so konfiguriert ist, dass er von einem Kliniker verwendet werden kann, um Hochdurchsatz-Sequenzierungsdaten, die von einem Patienten erhalten wurden, auf den Server (520) hochzuladen, wobei der Server (520) dazu konfiguriert ist,

den trainierten maschinellen Lernalgorithmus zu verwenden, um für jede Zelle der Sequenzierungsdaten ein Label "Responder" oder "Non-Responder" vorherzusagen, und
das Vorhersageergebnis einschließlich dasjenige/diejenigen Arzneimittel, das/die eine Reaktion hervorruft/hervorrufen, an den Client (540) zurückzugeben.

**10.** Verarbeitungssystem nach Anspruch 9, ferner umfassend einen Behandlungsplaner (550) als Frontend-Anwendung, der so konfiguriert ist, dass er von einem Kliniker verwendet werden kann, um das Vorhersageergebnis zu laden und eine Behandlung auszuwählen.

**11.** Nichtflüchtiges, computerlesbares Medium, das Code enthält, der dazu ausgelegt ist, ein oder mehrere Prozessoren eines Verarbeitungssystems zu veranlassen,

ein Genregulationsnetzwerk, GRN, und aus domänenspezifischem Wissen stammende Genannotationen zum Erzeugen eines multimodalen Wissensgraphen zu verwenden, wobei die Knoten des multimodalen Wissensgraphen Gene sind und wobei die Genannotationen die Beziehungen zwischen den Genen anreichern, wobei der multimodale Wissensgraph durch Kombinieren des GRN mit den Genannotationen zu einzelnen Vektordarstellungen der Gene des GRN erzeugt wird, welche Einbettungen der Gene des GRN darstellen;
eine Anzahl von Genmodulen, GMs, durch Clustern der Einbettungen der Gene des GRN zu erzeugen;
Proben von Hochdurchsatz-Sequenzierungsdaten in den multimodalen Wissensgraphen einzubetten, wobei die Sequenzierungsdaten Einzelzell-RNA-Sequenzierungsdaten umfassen, die durch Durchführung einer Einzelzell-RNA-Sequenzierung, scRNA-seq, an von einem Patienten entnommenen Proben erhalten werden;
für jede Probe der Sequenzierungsdaten einen Aktivierungsvektor zu erzeugen, wobei die jeweilige Probe als die Abstände zwischen der Einbettung und den Schwerpunkten jedes der Anzahl von GMs ausgedrückt wird;
die Aktivierungsvektoren als Eingabe zum Trainieren eines maschinellen Lernalgorithmus zur Vorhersage einer Reaktion einzelner Zellen auf ein interessierendes Arzneimittel zu verwenden;
den trainierten maschinellen Lernalgorithmus zu verwenden, um für jede Zelle der Sequenzierungsdaten ein Label "Responder" oder "Non-Responder" vorherzusagen; und
mittels eines Abstimmungsverfahrens die Gesamtreaktion des Patienten auf das betreffende Arzneimittel mit einem Konfidenzwert zu bestimmen, wobei der Anteil der als reagierend vorhergesagten Zellen die Konfidenz darstellt, dass der Patient auf das Arzneimittel ansprechen wird.

**Revendications**

**1.** Procédé mis en œuvre par ordinateur pour la stratification de patients à l'aide de données de séquençage d'ARN à haut débit, dans lequel les données de séquençage incluent des données de séquençage d'ARN monocellulaire obtenues en réalisant un séquençage d'ARN monocellulaire, scRNA-seq, d'échantillons prélevés sur un patient, le procédé comportant :

l'utilisation d'un réseau de régulation génique, GRN, et d'annotations géniques provenant de la connaissance de domaine pour générer un graphe de connaissances multimodal, dans lequel les nœuds du graphe de connaissances multimodal sont des gènes et dans lequel les annotations géniques enrichissent les relations entre les gènes, dans lequel le graphe de connaissances multimodal est généré en combinant le GRN avec les annotations géniques en représentations de vecteurs uniques des gènes du GRN, qui sont des intégrations des gènes du GRN ;

la création d'un certain nombre de modules de gènes, GM, en regroupant les intégrations des gènes du GRN ;

l'intégration des échantillons des données de séquençage dans le graphe de connaissances multimodal ;

la génération, pour chaque échantillon des données de séquençage, d'un vecteur d'activation dans lequel l'échantillon respectif est exprimé en tant que distances entre l'intégration et les centroïdes de chacun du nombre de GM ;

l'utilisation des vecteurs d'activation comme entrée pour entraîner un algorithme d'apprentissage automatique pour prédire une réponse de cellules individuelles à un médicament d'intérêt ;

l'utilisation de l'algorithme d'apprentissage automatique entraîné pour prédire une étiquette « répondeur » ou « non répondeur » pour chaque cellule des données de séquençage ; et

l'établissement par un processus de vote de la réponse totale du patient audit médicament d'intérêt avec un score de confiance, dans lequel la fraction de cellules prévue pour répondre audit médicament représente la confiance que le patient répondra audit médicament.

2. Procédé selon la revendication 1, dans lequel l'intégration des échantillons des données de séquençage dans le graphe de connaissances multimodal inclut la combinaison linéaire des données de séquençage avec les intégrations des gènes.

3. Procédé selon la revendication 1 ou 2, comportant en outre :

l'utilisation d'un réseau neuronal convolutif graphique, GCNN, pour créer les intégrations de chaque gène sur la base du GRN avec les annotations géniques ; et

le regroupement des intégrations et la fourniture des regroupements en tant que GM.

4. Procédé selon l'une quelconque des revendications 1 à 3, comportant en outre, avant la génération des vecteurs d'activation :

l'application d'un algorithme de filtrage collaboratif pour éliminer les valeurs de chute et d'autres sources de bruit des données de séquençage à haut débit.

5. Procédé selon l'une quelconque des revendications 1 à 4, comportant en outre :

la fourniture en sortie d'un ou plusieurs des vecteurs d'activation les plus importants utilisés dans la prédiction comme explication des résultats obtenus.

6. Système de traitement pour la stratification de patients à l'aide de données de séquençage d'ARN à haut débit, en particulier pour exécuter un procédé selon l'une quelconque des revendications 1 à 5, dans lequel les données de séquençage incluent des données de séquençage d'ARN monocellulaire obtenues par réalisation d'un séquençage d'ARN monocellulaire, scRNA-seq, d'échantillons prélevés sur un patient, le système comportant un ou plusieurs processeurs configurés pour

utiliser un réseau de régulation génique, GRN, et d'annotations géniques provenant de la connaissance de domaine pour générer un graphe de connaissances multimodal, dans lequel les nœuds du graphe de connaissances multimodal sont des gènes et dans lequel les annotations géniques enrichissent les relations entre les gènes, dans lequel le graphe de connaissances multimodal est généré en combinant le GRN avec les annotations géniques en représentations de vecteurs uniques des gènes du GRN, qui sont des intégrations des gènes du GRN ;

créer un certain nombre de modules de gènes, GM, en regroupant les intégrations des gènes du GRN ;

intégrer les échantillons des données de séquençage dans le graphe de connaissances multimodal ;

générer, pour chaque échantillon des données de séquençage, un vecteur d'activation dans lequel l'échantillon respectif est exprimé en tant que distances entre l'intégration et les centroïdes de chacun du nombre de GM ;

utiliser les vecteurs d'activation comme entrée pour entraîner un algorithme d'apprentissage automatique pour prédire une réponse de cellules individuelles à un médicament d'intérêt ;

utiliser l'algorithme d'apprentissage automatique entraîné pour prédire une étiquette « répondeur » ou « non répondeur » pour chaque cellule des données de séquençage ; et

établir, par un processus de vote, la réponse totale du patient audit médicament d'intérêt avec un score de confiance, dans lequel la fraction de cellules prévue pour répondre audit médicament représente la confiance que le patient répondra audit médicament.

7. Système de traitement selon la revendication 6, comportant en outre une base de données (510) contenant des échantillons à haut débit de différentes cellules tumorales, traités avec différents médicaments, ainsi que le graphe de connaissances multimodal.

8. Système de traitement selon la revendication 7, comportant en outre un serveur (520) configuré pour utiliser un graphe de connaissances multimodal de la base de données (510) pour entraîner un algorithme d'apprentissage automatique sur la base des vecteurs d'activation pour prédire une réponse de cellules individuelles à un médicament appliqué.

9. Système de traitement selon la revendication 8, comportant en outre un client (540) configuré pour être utilisé par un clinicien pour télécharger des données de séquençage à haut débit obtenues d'un patient sur le serveur (520), dans lequel le serveur (520) est configuré pour

   utiliser l'algorithme d'apprentissage automatique entraîné pour prédire une étiquette « répondeur » ou « non répondeur » pour chaque cellule des données de séquençage, et
   renvoyer le résultat de prédiction incluant le(s) médicament(s) qui donne(nt) une réponse au client (540).

10. Système de traitement selon la revendication 9, comportant en outre un planificateur de traitement (550) en tant qu'application frontale configurée pour être utilisée par un clinicien pour charger le résultat de prédiction et pour sélectionner un traitement.

11. Support non transitoire lisible par ordinateur comportant du code pour amener un ou plusieurs processeurs d'un système de traitement à :

   utiliser un réseau de régulation génique, GRN, et d'annotations géniques provenant de la connaissance de domaine pour générer un graphe de connaissances multimodal, dans lequel les nœuds du graphe de connaissances multimodal sont des gènes et dans lequel les annotations géniques enrichissent les relations entre les gènes, dans lequel le graphe de connaissances multimodal est généré en combinant le GRN avec les annotations géniques en représentations de vecteurs uniques des gènes du GRN, qui sont des intégrations des gènes du GRN ;
   créer un certain nombre de modules de gènes, GM, en regroupant les intégrations des gènes du GRN ;
   intégrer des échantillons de données de séquençage à haut débit dans le graphe de connaissances multimodal, dans lequel les données de séquençage incluent des données de séquençage d'ARN monocellulaire obtenues par la réalisation d'un séquençage d'ARN monocellulaire, scRNA-seq, d'échantillons prélevés sur un patient ;
   générer, pour chaque échantillon des données de séquençage, un vecteur d'activation dans lequel l'échantillon respectif est exprimé en tant que distances entre l'intégration et les centroïdes de chacun du nombre de GM ;
   utiliser les vecteurs d'activation comme entrée pour entraîner un algorithme d'apprentissage automatique pour prédire une réponse de cellules individuelles à un médicament d'intérêt ;
   utiliser l'algorithme d'apprentissage automatique entraîné pour prédire une étiquette « répondeur » ou « non répondeur » pour chaque cellule des données de séquençage ; et
   établir, par un processus de vote, la réponse totale du patient audit médicament d'intérêt avec un score de confiance, dans lequel la fraction de cellules prévue pour répondre audit médicament représente la confiance que le patient répondra audit médicament.

100

Start

S110 — Identification of Gene Clusters from external information embeddings

S120 — Imputation of dropout expression values

S130 — Representation of individual cells

# Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

| GM 0 | GM 1 | GM 2 | GM 3 |
|---|---|---|---|
| GO:0000278 (mitotic cell cycle) | GO:0003723 (RNA binding) | GO:0002376 (immune system process) | GO:0005783 (endoplasmic reticulum) |
| GO:0005634 (nucleus) | GO:0004518 (nuclease activity) | GO:0005576 (extracellular region) | GO:0006457 (protein folding) |
| GO:0005737 (cytoplasm) | GO:0005654 (nucleoplasm) | GO:0005615 (extracellular space) | GO:0006605 (protein targeting) |
| GO:0005815 (microtubule organizing center) | GO:0005730 (nucleolus) | GO:0005764 (lysosome) | GO:0006810 (transport) |
| GO:0007049 (cell cycle) | GO:0006259 (DNA metabolic process) | GO:0005773 (vacuole) | GO:0006914 (autophagy) |
| GO:0008283 (cell population proliferation) | GO:0006399 (tRNA metabolic process) | GO:0005886 (plasma membrane) | GO:0006950 (response to stress) |
| GO:0016301 (kinase activity) | GO:0006913 (nucleocytoplasmic transport) | GO:0005975 (carbohydrate metabolic process) | GO:0007034 (vacuolar transport) |
| GO:0016791 (phosphatase activity) | GO:0009058 (biosynthetic process) | GO:0006629 (lipid metabolic process) | GO:0007165 (signal transduction) |
| GO:0030154 (cell dierentiation) | GO:0016874 (ligase activity) | GO:0006810 (transport) | GO:0009056 (catabolic process) |
| GO:0048856 (anatomical structure development) | GO:0034641 (cellular nitrogen compound metabolic process) | GO:0007155 (cell adhesion) | GO:0015031 (protein transport) |
| GO:0051301 (cell division) | GO:0034655 (nucleobase-containing compound catabolic process) | GO:0016192 (vesicle-mediated transport) | GO:0016192 (vesicle-mediated transport) |
| | GO:0051301 (cell division) | GO:0031012 (extracellular matrix) | GO:0031410 (cytoplasmic vesicle) |
| | | GO:0040011 (locomotion) | GO:0042592 (homeostatic process) |
| | | GO:0042592 (homeostatic process) | GO:0051082 (unfolded protein binding) |
| | | GO:0048870 (cell motility) | |

# Fig. 14a

| GM 4 | GM 5 | GM 6 | GM 7 |
|---|---|---|---|
| GO:0003723 (RNA binding) | GO:0003723 (RNA binding) | GO:0005739 (mitochondrion) | GO:0003924 (GTPase activity) |
| GO:0003735 (structural constituent of ribosome) | GO:0006464 (cellular protein modication process) | GO:0006091 (generation of precursor metabolites and energy) | GO:0005768 (endosome) |
| GO:0005198 (structural molecule activity) | GO:0008135 (translation factor activity, RNA binding) | GO:0016491 (oxidoreductase activity) | GO:0005794 (Golgi apparatus) |
| GO:0005739 (mitochondrion) | GO:0022618 (ribonucleoprotein complex assembly)) | GO:0022857 (transmembrane transporter activity) | GO:0005856 (cytoskeleton) |
| GO:0005840 (ribosome) | | GO:0044281 (small molecule metabolic process) | GO:0005886 (plasma membrane) |
| GO:0006412 (translation) | | GO:0051186 (obsolete cofactor metabolic process) | GO:0006810 (transport) |
| GO:0009058 (biosynthetic process) | | GO:0055085 (transmembrane transport) | GO:0007010 (cytoskeleton organization) |
| GO:0016779 (nucleotidyltransferase activity) | | GO:0065003 (protein-containing complex assembly) | GO:0007155 (cell adhesion) |
| GO:0019843 (rRNA binding) | | | GO:0007165 (signal transduction) |
| GO:0032991 (protein-containing complex) | | | GO:0008092 (cytoskeletal protein binding) |
| GO:0034641 (cellular nitrogen compound metabolic process) | | | GO:0015031 (protein transport) |
| | | | GO:0016192 (vesicle-mediated transport) |
| | | | GO:0030705 (cytoskeletondependent intracellular transport) |
| | | | GO:0031410 (cytoplasmic vesicle) |
| | | | GO:0034330 (cell junction organization) |
| | | | GO:0040011 (locomotion) |
| | | | GO:0048870 (cell motility) |
| | | | GO:0061024 (membrane organization) |

## Fig. 14b

| GM 8 | GM 9 |
|---|---|
| GO:0000228 (nuclear chromosome) | GO:0003700 (DNA-binding transcription factor activity) |
| GO:0003674 (molecular function) | GO:0006629 (lipid metabolic process) |
| GO:0003677 (DNA binding) | |
| GO:0003723 (RNA binding) | |
| GO:0003729 (mRNA binding) | |
| GO:0004386 (helicase activity) | |
| GO:0005634 nucleus) | |
| GO:0005654 (nucleoplasm) | |
| GO:0005694 (chromosome) | |
| GO:0006259 (DNA metabolic process) | |
| GO:0006397 (mRNA processing) | |
| GO:0006913 (nucleocytoplasmic transport) | |
| GO:0016887 (ATPase activity) | |
| GO:0032991 (protein-containing complex) | |
| GO:0034641 (cellular nitrogen compound metabolic process) | |
| GO:0051276 (chromosome organization) | |

## Fig. 14c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **D. VAN DIJK et al.** Recovering gene interactions from single-cell data using data diffusion. *Cell*, 2018, vol. 174 (3), 716-72927
- **M. HUANG et al.** SAVER: gene expression recovery for single-cell RNA sequencing. *Nature Methods*, 2018, vol. 15, 539-542
- **W.V. LI et al.** An accurate and robust imputation method scImpute for single-cell RNA-seq data. *Nature Communications*, 2018, vol. 9 (997)
- **G. ERASLAN et al.** Single-cell RNA-seq denoising using a deep count autoencoder. *Nature Communications*, 2019, vol. 10 (390)
- **J. RAO et al.** Imputing Single-cell RNA-seq data by combining Graph Convolution and Autoencoder Neural Networks. *bioRxiv*, 02 May 2020, 935296
- **X. LI et al.** Deep learning enables accurate clustering with batch effect removal in single-cell RNA-seq analysis. *Nature Communications*, 2020, vol. 11 (2338)
- **SHARMA et al.** Longitudinal single-cell RNA sequencing of patient-derived primary cells reveals drug-induced infidelity in stem cell hierarchy. *Nature Communications*, 2018, vol. 9 (4931)
- **J. FAN, J et al.** Characterizing transcriptional heterogeneity through pathway and gene set over-dispersion analysis. *Nature Methods*, 2016, vol. 13, 241-244
- **J. MA et al.** Using deep learning to model the hierarchical structure and function of a cell. *Nature Methods*, 2018, vol. 15, 290-298
- **F.G. FROST et al.** Pan-cancer RNA-seq data stratifies tumours by some hallmarks of cancer. *Journal of Cellular and Molecular Medicine*, 2020, vol. 24, 418-430
- **B. SHICKEL et al.** Deep EHR: A survey of recent advances in deep learning techniques for electronic health record (EHR) analysis. *IEEE Journal of Biomedical and Health Informatics*, 2018, vol. 22 (5), 1589-1605
- **D. TORO-DOMÍNGUEZ et al.** Differential treatments based on drug-induced gene expression signatures and longitudinal systemic lupus erythematosus stratification. *Scientific Reports*, 2019, vol. 9 (15502)
- **FRÖHLICH et al.** Premenopausal breast cancer: potential clinical utility of a multi-omics based machine learning approach for patient stratification. *EPMA Journal*, 2018, vol. 9, 175-186
- **P. JEYANANTHAN et al.** Classification and regression analysis of lung tumors from multi-level gene expression data. *Proceedings of the International Joint Conference on Neural Networks*, 2019
- **Y. JANG et al.** CaPSSA: visual evaluation of cancer biomarker genes for patient stratification and survival analysis using mutation and expression data. *Bioinformatics*, 2019, vol. 35 (24), 5341-5343
- **F.J. CAMPOS-LABORIE et al.** DECO: decompose heterogeneous population cohorts for patient stratification and discovery of sample biomarkers using omic data profiling. *Bioinformatics*, 2019, vol. 35 (19), 3651-3662
- **L. YU**. RNA-Seq reproducibility assessment of the sequencing quality control project. *Cancer informatics*, 2020, vol. 19, 1-5
- **K.-T. KIM et al.** Single-cell mRNA sequencing identifies subclonal heterogeneity in anti-cancer drug responses of lung adenocarcinoma cells. *Genome Biology*, 2015, vol. 16 (127)
- **KOTNIS, B** ; **NASTASE, V**. Analysis of the impact of negative sampling on link prediction in knowledge graphs. KnoProceedings of the 1st Workshop on Knowledge Base Construction. *Reasoning and Mining*, 2018
- Integrating single-cell transcriptomic data across different conditions, technologies, and species. *Nature Biotechnology*, 2018, vol. 36 (5), 411-420
- **HUG, N**. Surprise: A python library for recommender systems. *Journal of Open Source Software*, 2020, vol. 5 (2174)
- **RAJKUMAR, S.V.** Multiple myeloma: 2012 update on diagnosis, risk-stratification, and management. *American Journal of Hematology*, 2012, vol. 87, 77-88
- **MCINNES, L** ; **HEALY, J** ; **MELVILLE, J**. *UMAP: Uniform Manifold Approximation and Projection for Dimension Reduction*, 2018
- **M.T. RIBEIRO et al.** why should i trust you?": Explaining the predictions of any classifier. *Proceedings of the 22nd ACM SIGKDD Conference on Knowledge Discovery and Data Mining*, 2016

- **TANAKA, K**. The proteasome: Overview of structure and functions. *Proceedings of the Japan Academy, Series B*, 2009, vol. 85, 12-36